# FASCICULE DE BREVET EUROPEEN

(11) **EP 2 661 502 B1**
(45) Date de publication et mention de la délivrance du brevet: **09.03.2016**
(21) Numéro de dépôt: 12700381.2
(22) Date de dépôt: 05.01.2012
(51) Int. Cl.: C12Q 1/37, G01N 33/543, G01N 33/573, G01N 33/574, C07K 17/14

(54) **BIOCAPTEUR POUR LA DETECTION DE LA PRESENCE DE PROTEASES ET EVENTUELLEMENT LA QUANTIFICATION DE L'ACTIVITE ENZYMATIQUE DE CELLES-CI**
BIOSENSOR ZUM NACHWEIS VON PROTEASEN UND ZUR OPTIONALEN QUANTIFIZIERUNG DER ENZYMATISCHEN AKTIVITÄT DAVON
BIOSENSOR FOR DETECTING THE PRESENCE OF PROTEASES AND OPTIONALLY QUANTIFYING THE ENZYMATIC ACTIVITY THEREOF

(30) Priorité: 06.01.2011 FR 1150100
(43) Date de publication de la demande: 13.11.2013
(73) Titulaire: Commissariat à l'Énergie Atomique et aux Énergies Alternatives, 75015 Paris (FR)
(72) Inventeur: ALOUINI, Mohamed Anis, Sousse 4000 (TN); BERTHELOT, Thomas, F-91940 Les Ulis (FR); MOUSTOIFA, El Farouck, F-97610 Labattoir-mayotte (FR); ALBENQUE-RUBIO, Sandra, F-33127 Martignas Sur Jalle (FR); DELERIS, Gérard, F-33000 Bordeaux (FR)
(74) Mandataire: Ahner, Philippe
(86) Numéro de dépôt international: PCT/EP2012/050158
(87) Numéro de publication internationale: WO 2012/093162

(56) Documents cités:
- WO-A1-2010/000593
- WO-A2-02/100801
- US-A1- 2006 160 207
- OH SANG WOOK ET AL: "Calixarene derivative as a tool for highly sensitive detection and oriented immobilization of proteins in a microarray format through noncovalent molecular interaction", FASEB JOURNAL, FED. OF AMERICAN SOC. FOR EXPERIMENTAL BIOLOGY, US, vol. 19, no. 10, 1 août 2005 (2005-08-01), pages 1335-1337, XP002491453, ISSN: 0892-6638
- HUANG F ET AL: "Cucurbiturils: Molecular Nanocapsules for Time-Resolved Fluorescence-Based Assays", IEEE TRANSACTIONS ON NANOBIOSCIENCE, IEEE SERVICE CENTER, PISCATAWAY, NY, US, vol. 3, no. 1, 1 mars 2004 (2004-03-01), pages 39-45, XP011108764, ISSN: 1536-1241, DOI: 10.1109/TNB.2004.824269
- REYNALDO VILLALONGA ET AL: "Supramolecular Chemistry of Cyclodextrins in Enzyme Technology", CHEMICAL REVIEWS, vol. 107, no. 7, 1 juillet 2007 (2007-07-01), pages 3088-3116, XP055006192, ISSN: 0009-2665, DOI: 10.1021/cr050253g

## Description

### DOMAINE TECHNIQUE

La présente invention a trait à un biocapteur réutilisable après emploi, permettant de déterminer la présence et/ou quantifier l'activité enzymatique de protéases, en particulier, de métalloprotéases, telles que des enzymes métalloprotéases de matrice extracellulaire (connus sous l'abréviation MMP).

Ces biocapteurs trouvent leur application dans le domaine du diagnostic, notamment dans la détermination de l'état d'avancement de certaines pathologies impliquant des protéases (telles que le cancer, et particulièrement l'angiogénèse tumorale et l'agressivité tumorale), grâce à la possibilité de pouvoir quantifier l'activité des protéases directement liée à la pathologie et dans le domaine du développement et de la validation de nouvelles thérapeutiques contre ces protéases, par exemple, par la mise en place de nouvelles molécules présentant une activité inhibitrice vis-à-vis de ces enzymes.

### ÉTAT DE LA TECHNIQUE ANTÉRIEURE

En particulier, les protéases du type MMP sont une famille d'endopeptidases, au nombre de 28, contenant un atome de zinc au niveau de leur site actif. Elles peuvent être classées en deux sous-familles, selon qu'elles sont sécrétées ou associées à une membrane. Les MMP sécrétées comprennent les collagénases (telles que MMP-1, 8, 13 et 18), les gélatinases (telles que MMP-2 et 9), les matrilysines (telles que MMP-7 et 26), les stromélysines (telles que MMP-3, 10 et 11), l'épilysine (MMP-28), l'énamélysine (MMP-20). Les MMP associées à une membrane comprennent les MMP transmembranaires telles que MMP-14, MMP-15, MMP-16, MMP-17, MMP-23, MMP-24 et MMP-25.

Les MMP sont capables de cliver tous les éléments de la matrice extracellulaire ainsi que certains facteurs de croissance, ou encore des molécules d'adhésion. Les MMP sont régulées par des inhibiteurs naturels, tels que les TIMP (abréviation correspondant aux inhibiteurs tissulaires de métalloprotéases), et jouent un rôle important dans les processus physiologiques (tels que la croissance embryonnaire, l'angiogénèse, le remodelage du tissu osseux). Lorsque la balance MMP/TIMP est déréglée, des processus pathologiques apparaissent tels que la croissance tumorale, les métastases, les arthrites rhumatoïdes, les maladies cardiaques.

Le rôle des MMP dans la progression tumorale est multiple, notamment du fait de la capacité des MMP à dégrader la matrice extracellulaire, favorisant ainsi l'invasion tumorale. Il a été démontré que les MMP ont également une activité protéolytique contre des protéines non matricielles, ce qui leur confère un rôle complexe dans plusieurs autres étapes de la progression tumorale, la perte d'adhérence, l'invasion, la prolifération, l'angiogénèse, l'intravasion, l'extravasion et la croissance métastatique.

La cellule endothéliale présente une remarquable stabilité génétique et sera donc peu sujette à des mutations provoquant une résistance aux traitements. L'angiogénèse tumorale est donc clairement l'un des domaines les plus prometteurs et les plus actifs de la recherche contre le cancer. Plusieurs produits actuellement utilisés pour le traitement de certaines tumeurs ont des effets anti-angiogéniques.

Le remodelage de la matrice extracellulaire, en particulier sa dégradation, est nécessaire dans plusieurs étapes de la progression tumorale. Cette dégradation est assurée en grande partie par les MMP.

L'expression des MMP varie selon les étapes de la progression tumorale. Par exemple, ce sont les gélatinases (MMP-2 et 9), qui sont les plus exprimées dans l'angiogénèse tumorale. La plupart de ces MMP, en l'occurrence MMP-1, 2, 3, 7 et 8, 9, 10 et 13 et la MT1-MMP sont devenues des cibles thérapeutiques importantes pour le traitement de tumeurs cancéreuses.

Les MMP sont impliquées dans la dégradation de nombreuses protéines de la matrice extracellulaire mais aussi non matricielles.

De par leur implication dans de nombreux mécanismes biologiques et en particulier, dans des processus tumoraux, les enzymes du type MMP sont devenues de véritables cibles thérapeutiques en vue de lutter contre le cancer, en particulier dans le but d'inhiber directement la croissance tumorale mais aussi d'inhiber la formation de métastases.

Afin de comprendre le mécanisme d'action tant au niveau extra- que péricellulaire, leur régulation de sorte à pouvoir accéder à des nouveaux traitements chimiothérapeutiques et/ou radiothérapeutiques, il importe de disposer de systèmes permettant de :
- tester l'activité inhibitrice de nouvelles molécules vis-à-vis d'enzymes MMP ;
- quantifier l'expression et l'activité des MMP, qui peuvent être liées à l'agressivité des tumeurs ;
- localiser l'activité des MMP lors de la polarisation cellulaire.

Actuellement, pour les protéases telles que les MMP, ces tests en solution utilisent des substrats synthétiques modifiés ou des substrats naturels pour doser l'activité protéasique, par exemple, par immunofluorescence. Toutefois, il est à noter que ces substrats présentent des problèmes de solubilité et de concentration lors des mesures en milieu liquide.

Une idée pour contourner ces difficultés consisterait à fixer ces substrats sur un support solide (de sorte à former un biocapteur), ce qui pourrait permettre d'augmenter la fiabilité des résultats et également d'aller vers une automatisation des tests, afin de minimiser la quantité utilisée de substrat.
Le document WO2010/000593 décrit un biocapteur pour la détection de l'activité enzymatique d'une protéase comprenant un support fonctionnalisé par des composés silane formant une liaison covalent avec ledit support et comprenant un bras espaceur, sur lequel est greffé le substrat de la protéase.

Le document Sang Wook Oh et al, The FASEB Journal, vol.19, no 10, 2005, décrit quant à lui le concept de biocapteurs fonctionnalisés par des composés organiques comprenant au moins un groupe formant cage, ie des calixarènes, pour fixer des protéines telles que des anticorps ou encore des enzymes.

Les auteurs de la présente invention se sont ainsi fixé pour objectif de proposer de tels biocapteurs pouvant être utilisés sous forme de plateformes pour déterminer la présence de protéases, telles que les enzymes MMP et/ou quantifier ou localiser l'activité enzymatique de ces protéases, ce biocapteur devant permettre la transduction d'un signal d'activité.

Qui plus est, les auteurs de la présente invention se sont fixé pour objectif de proposer des biocapteurs, qui soient réutilisables après emploi, ce qui signifie, en d'autres termes, qu'ils puissent être régénérés, après une opération de détermination de la présence et/ou de quantification de l'activité enzymatique d'une protéase donnée, en vue de pouvoir être réutilisés pour d'autres opérations ultérieures de ce type.

### EXPOSÉ DE L'INVENTION

Ainsi, les auteurs de la présente invention ont conçu un nouveau biocapteur pour la détection de la présence et/ou de l'activité enzymatique d'au moins une protéase, qui correspond à un biocapteur comprenant un support fonctionnalisé par des composés organiques comprenant au moins un groupe formant cage, lequel groupe formant cage est complexé avec au moins un groupe d'un composé comprenant une séquence peptidique cible de la ou les protéases dont on veut déterminer la présence et/ou l'activité enzymatique.

Avant d'entrer plus en détail dans la présente description, nous proposons les définitions suivantes.

Par séquence peptidique cible de la ou les protéases dont on veut déterminer la présence et/ou l'activité enzymatique, on entend, classiquement, une séquence peptidique apte à être reconnue et clivée par la ou lesdites protéases susmentionnées.

Par support fonctionnalisé, on entend un support auquel sont liés, par exemple par le biais de liaison de covalence, des composés organiques tels que définis ci-dessus, à savoir des composés organiques comprenant au moins un groupe formant cage.

Sans que l'on soit nullement lié par la théorie, le composé comprenant une séquence peptidique cible de la ou les protéases, dont on veut déterminer la présence et/ou l'activité enzymatique, forme, par le biais d'au moins un de ses groupes, avec le groupe formant cage (issu d'une molécule cage) un complexe du type « hôte-invité » de par la nature intrinsèque des groupes formant cage à former une cavité hydrophobe qui va piéger ledit composé par affinité chimique.

Le support susmentionné peut être un support solide inorganique.

Par exemple, les supports solides inorganiques peuvent comprendre un matériau choisi dans le groupe constitué par les verres, les quartz, les céramiques (telles que les céramiques oxydes, comme la silice, l'alumine), les métaux (tels que l'aluminium, le chrome, le cuivre, le zinc, l'argent, le nickel, l'étain, l'or ou les mélanges de ceux-ci) ou les métalloïdes (tels que le silicium ou le germanium).

Par exemple, le support peut être un support comprenant de l'or éventuellement allié avec un autre métal, l'or éventuellement allié pouvant se présenter sous forme d'une couche de revêtement recouvrant une plaque en verre.

Par exemple, le support solide peut être, également, un support en silicium, ce support pouvant être éventuellement oxydé, moyennent quoi il comportera, à sa surface, des groupes hydroxyles aptes à permettre sa fonctionnalisation par greffage de composés organiques tels que définis ci-dessus.

Le support peut également être de nature organique. Il peut s'agir notamment d'un support en un polymère choisi parmi les polyuréthanes, les polyoléfines, les polycarbonates, les polyéthylènetéréphtalates, ces polymères étant avantageusement fluorés voire perfluorés. Parmi les polymères fluorés susceptibles de convenir, on peut citer le polyfluorure de vinylidène (connu sous l'abréviation PVDF), les copolymères de tétrafluoroéthylène et de tétrafluoropropylène (connus sous l'abréviation FEP), les copolymères d'éthylène et de tétrafluoroéthylène (connus sous l'abréviation ETFE), les copolymères d'hexafluoropropène et fluorure de vinylidène (connus sous l'abréviation HFP-co-VDF).

De préférence, le support est en polyfluorure de vinylidène.

Les composés organiques susmentionnés peuvent consister en un bras espaceur lié par une des ses extrémités au support et par au moins une autre de ses extrémités à au moins un groupe formant cage.

Le groupe formant cage peut être un groupe calixarène, un groupe cucurbituril ou un groupe β-cyclodextrine.

Avantageusement, le groupe formant cage est un groupe β-cyclodextrine.

Sans être lié par la théorie, un groupe β-cyclodextrine forme, de façon intrinsèque, une cage hydrophobe présentant la capacité à se complexer à des groupes appartenant, dans notre cas, aux composés comprenant une séquence peptidique cible de la protéase dont on veut déterminer la présence et/ou l'activité enzymatique, ces groupes pouvant être des groupes benzéniques, des groupes adamantanes.

Le groupe β-cyclodextrine est un groupe cyclique pouvant comprendre, dans son cycle, au moins un motif répétitif de formule (I) suivante : 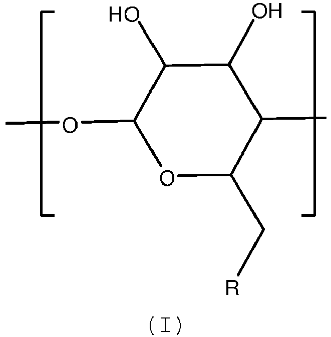 dans laquelle R représente un groupe hydroxyle ou un groupe amino,
et au moins un motif de formule (II) suivante : 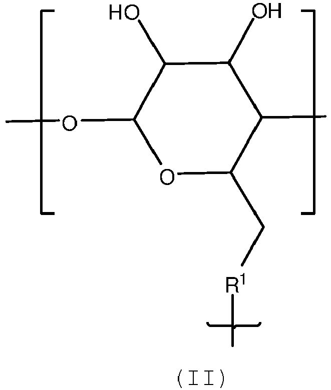 R¹ correspondant à un atome d'oxygène ou à un groupe amino,
l'accolade indiquant l'endroit par lequel le groupe β-cyclodextrine est lié au support via éventuellement un groupe espaceur.

Le groupe β-cyclodextrine peut correspondre à un enchaînement de 7 motifs, dont 6 motifs répondent à la formule (I) telle que mentionnée ci-dessus et un seul motif correspond à la formule (II) telle que mentionnée ci-dessus, ce dernier faisant le lien avec le support via éventuellement un groupe espaceur.

Un tel groupe β-cyclodextrine peut ainsi répondre à la formule (III) suivante : 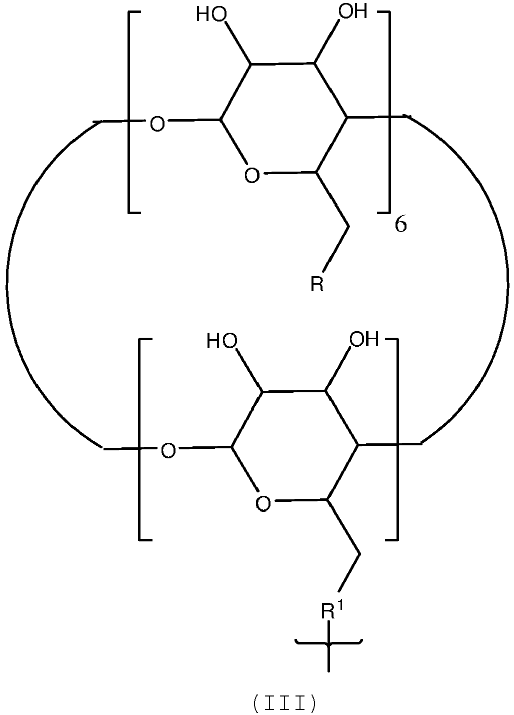 dans laquelle R et R¹ répondent aux mêmes définitions que celles données ci-dessus.

L'éventuel groupe espaceur faisant le lien entre le support et le groupe formant cage peut être un groupe hydrocarboné, sous forme d'une chaîne linéaire ou ramifiée, pouvant comporter un ou plusieurs groupes aromatiques et pouvant comporter un ou plusieurs hétéroatomes, tels que O, S et N.

Les groupes hydrocarbonés susmentionnés constitutifs du groupe espaceur peuvent être de divers types.

Ainsi, selon une première variante, ils peuvent être constitués d'une chaîne hydrocarbonée linéaire, par exemple, une chaîne alkyle comprenant de 1 à 25 atomes de carbone, la chaîne hydrocarbonée pouvant être terminée par un groupe terminal non hydrocarboné, tel qu'un groupe -CO-, assurant la jonction avec le groupe formant cage.

Un exemple spécifique, conforme à cette première variante, peut être un groupe répondant à la formule (IV) suivante :

-(CH₂)₁₀-CO- (IV)

Selon une deuxième variante, ils peuvent être constitués d'une chaîne hydrocarbonée linéaire interrompue par un ou plusieurs hétéroatomes, tels que O, N et/ou S (et plus particulièrement par un ou plusieurs atomes d'oxygène), ladite chaîne hydrocarbonée pouvant être terminée par un groupe terminal non hydrocarboné, tel qu'un groupe -CO-, assurant la jonction avec le groupe formant cage.

Un exemple spécifique, conforme à cette deuxième variante, peut être un groupe répondant à la formule (V) suivante :

- (CH₂)₂₂-O-(CH₂-O)₃-CH₂-CH₂-CO- (V)

Selon une troisième variante, ils peuvent être constitués d'une chaîne hydrocarbonée ramifiée comportant une chaîne principale hydrocarbonée et un ou plusieurs groupes pendants à ladite chaîne principale, ce ou ces groupes pendants assurant la liaison avec le ou les groupes formant cage, ce ou ces groupes pendants pouvant être un groupe -CO-, ladite chaîne principale pouvant être liée directement au support ou via un groupe formant pont, tel qu'un groupe phényle.

Un exemple spécifique, conforme à cette troisième variante, peut être un groupe de formule (VI) suivante : 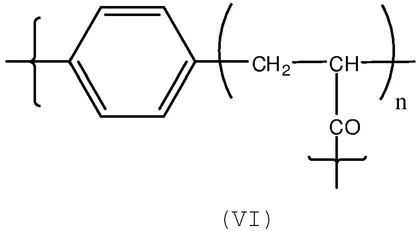 n correspondant au nombre de répétition du motif pris entre parenthèses, l'accolade au niveau du groupe phényle indiquant l'endroit par lequel le groupe espaceur est lié au support et l'accolade au niveau du groupe -CO- indiquant l'endroit par lequel le groupe espaceur est lié à un groupe formant cage.

Des supports fonctionnalisés spécifiques conformes à l'invention peuvent être :
- des supports en silicium fonctionnalisés par des composés organiques consistant en un bras espaceur de formule -(CH₂)₁₀-CO- lié au support par le groupement terminal -CH₂- et lié à un groupe β-cyclodextrine de formule (III) mentionnée ci-dessus par le groupement -CO- ;
- des supports en silicium fonctionnalisés par des composés organiques consistant en un bras espaceur de formule -(CH₂)₂₂-O-(CH₂-O)₃-CH₂-CH₂-CO- lié au support par le groupement terminal -CH₂- et lié à un groupe β-cyclodextrine de formule (III) mentionnée ci-dessus par le groupement -CO- ;
- des supports comprenant de l'or éventuellement allié à un autre métal fonctionnalisés par des composés organiques consistant en un bras espaceur de formule (VI) susmentionnée lié au support par le biais du groupement terminal phényl et lié à des groupes β-cyclodextrine de formule (III) mentionnée ci-dessus par les groupements -CO-.

Comme mentionné ci-dessus, les groupes formant cage forment un complexe avec des groupes appartenant à un composé comprenant une séquence peptidique cible de la ou les protéases dont on veut déterminer la présence et/ou l'activité enzymatique.

La ou les protéases dont on veut déterminer l'activité enzymatique peuvent être des enzymes du type MMP, telles que des enzymes MMP-1, MMP-2, MMP-3, MMP-5, MMP-7, MMP-8, MMP-9, MMP-10, MMP-11, MMP-12, MMP-13, MMP-14, MMP-15, MMP-16, MMP-17, MMP-19, MMP-23, MMP-25 et MMP-26

Les séquences peptidiques cibles peuvent être issues de séquences peptidiques naturelles rencontrées dans les substrats naturels des MMP ou bien être issues de séquences synthétiques reconnues comme étant des substrats synthétiques des enzymes MMP.

Dans certains cas, il est possible qu'un substrat naturel ou synthétique soit reconnu et clivé par plusieurs enzymes MMP. Afin de déterminer quelle enzyme MMP est responsable du clivage, il faudra alors utiliser et comparer les données de cinétique enzymatique des différentes enzymes MMP pour ce substrat.

Ces données de cinétique enzymatique qui permettent la discrimination entre plusieurs enzymes MMP, sont généralement la constante de Mickaelis (Km) qui est l'indicateur d'affinité d'une enzyme pour son substrat et le rapport de la constante catalytique (Kcat, nombre de moles de produits formées par seconde et par mole d'enzyme) sur la constante de Mickaelis qui est l'indicateur de l'efficacité catalytique de l'enzyme. Généralement, il existe donc un couple de valeurs (Km et kcat/Km) spécifique pour un couple MMP/substrat. Il sera donc possible, à titre d'exemple, pour 2 enzymes MMP reconnaissant le même substrat de déterminer laquelle est responsable de l'activité visualisée.

Les séquences synthétiques peuvent comprendre, par exemple les acides aminés ou groupes suivants :
Nva (Nor-valine) ; Cha (3-cyclohexylalanine) ; Dpa (N-3(2,4-dinitrophényl)-L-2,3-diaminopropionyle) ; Abu : acide α-aminobutyrique ; Cys(Me) correspondant à la S-méthylcystéine, ce qui signifie que le groupe -SH est remplacé par le groupe-S-CH₃, D-Arg correspondant à la D-Arginine.

Dans la suite de cet exposé, on précise que les abréviations listées ci-dessous ont les significations suivantes :
Gly : glycine ; Pro : proline ; Leu : leucine ; Alla : alanine ; Gln : glutamine ; Ile : isoleucine ; Arg : arginine ; Val : valine ; Tyr : tyrosine ; Glu : glutamate ; Met : méthionine ; Phe : phénylalanine ; Asn : Asparagine ; Thr : thréonine ; Trp : tryptophane ; Ser : sérine ; His : histidine, ces abréviations correspondant à la nomenclature officielle à 3 lettres des acides aminés.

Lorsque la séquence peptidique cible est destinée à être cible de l'enzyme MMP-1, elle peut ainsi correspondre à l'une des séquences suivantes :
-Gly-Pro-Gln-Gly-Leu-Leu-Gly-Ala-
   SEQ. 1
-Ala-Pro-Gln-Gly-Ile-Ala-Gly-Gln-
   SEQ. 2
-Gly-Pro-Gln-Gly-Leu-Ala-Gly-Gln-
   SEQ.3
-Gly-Pro-Leu-Gly-Ile-Ala-Gly-Ile -
   SEQ.4
-Gly-Pro-Glu-Gly-Leu-Arg-Val-Gly-
   SEQ.5
-Tyr-Glu-Ala-Gly-Leu-Gly-Val-Val-
   SEQ.6
-Ala-Gly-Leu-Gly-Val-Val-Glu-Arg-
   SEQ.7
-Ala-Gly-Leu-Gly-Ile-Ser-Ser-Thr-
   SEQ.8
-Gly-Ala-Met-Phe-Leu-Glu-Ala-Ile-
   SEQ.9
-Ile-Pro-Glu-Asn-Phe-Phe-Gly-Val-
   SEQ.10
-Thr-Glu-Gly-Glu-Ala-Arg-Gly-Ser-
   SEQ.11
*-Arg-Ala-Ile-His-Ile-Gln-Ala-Glu-*
   *SEQ.12*
*-Leu-Arg-Ala-Tyr-Leu-Leu-Pro-Ala-*
   *SEQ.13*
*-Gly-Pro-Leu-Gly-Met-Arg-Gly-Leu-*
   *SEQ.14*
*-Pro-Gln-Gly-Leu-Glu-Ala-Lys-*
   *SEQ.15*
*-Pro-Leu-Gly-Leu-Trp-Ala-D-Arg-*
   *SEQ. 16*
*-Pro-Leu-Ala-Leu-Trp-Ala-Arg-*
   *SEQ. 1*7
-Pro-Cha-Abu-Cys(Me)-His-Ala-
   SEQ.18
-Pro-Cha-Gly-Cys(Me)-His-Ala-
   SEQ. 19
*-Pro-Cha-Gly-Cys(Me)-His-Ala-*
   *SEQ. 20*
*-Pro-Leu-Gly-Leu-Dpa-Ala-Arg-*
   *SEQ.21*
*-Pro-Leu-Gly-[2-mercapto-4-methyl-pentanoyl]-Leu-Gly-*
   *SEQ. 22*
*-Pro-Leu-Ala-Leu-Trp-Ala-Arg-*
   *SEQ. 1*7
*-Pro-Leu-Gly-Cys(Me)-His-Ala-D-Arg-*
   *SEQ. 23*
*-Arg-Pro-Leu-Ala-Leu-Trp-Arg-*
   *SEQ. 24*
*-Pro-Cha-Gly-Nva-His-Ala-*
   *SEQ. 25*
*-Arg-Pro-Lys-Pro-Tyr-Ala-Nva-Trp-Met-*
   *SEQ. 26*

Lorsque la séquence peptidique cible est destinée à être cible de l'enzyme MMP-2, elle peut ainsi correspondre à l'une des séquences suivantes :
*-Ile-Pro-Glu-Asn-Phe-Phe-Gly-Val-*
   *SEQ.10*
-Pro-Pro-Gly-Ala-Tyr-His-Gly-Ala-
   SEQ.27
*-Arg-Ala-Ile-His-Ile-Gln-Ala-Glu-*
   *SEQ.12*
*-Gly-Pro-His-Leu-Leu-Val-Glu-Ala-*
   *SEQ.28*
*-Leu-Arg-Ala-Tyr-Leu-Leu-Pro-Ala-*
   *SEQ.13*
-Pro-Gln-Gly-Leu-Glu-Ala-Lys-
   SEQ.29
*-Pro-Leu-Gly-Leu-Trp-Ala-D-Arg-*
   *SEQ. 16*
-Pro-Leu-Ala-Nva-Dpa-Ala-Arg-
   SEQ.30
*-Pro-Leu-Gly-Leu-Dpa-Ala-Arg-*
   *SEQ.21*
*-Pro-Leu-Gly-Met-Trp-Ser-Arg-*
   *SEQ. 31*
*-Pro-Leu-Gly-SCH[CH₂CH (CH₃) ₂]-CO-Leu-Gly-*
   *SEQ. 32*
*-Arg-Pro-Pro-Gly-Phe-Ser-Ala-Phe-*
   *SEQ. 33*
*-Pro-Leu-Gly-Leu-Dpa-Ala-Arg-*
   *SEQ.21*
*-Pro-Leu-Gly-[2-mercapto-4-methyl-pentanoyl]-Leu-Gly-*
   *SEQ.22*
*-Pro-Leu-Gly-Cys(Me)-His-Ala-D-Arg-*
   *SEQ.23*
*-Arg-Pro-Leu-Ala-Leu-Trp-Arg-*
   *SEQ.24*
*-Pro-Cha-Gly-Nva-His-Ala-*
   *SEQ. 25*
*-Arg-Pro-Lys-Pro-Tyr-Ala-Nva-Trp-Met-*
   *SEQ. 26*

Lorsque la séquence peptidique cible est destinée à être cible de l'enzyme MMP-3, elle peut ainsi correspondre à l'une des séquences suivantes :
- *Gly-Pro-Glu-Gly-Leu-Arg-Val-Gly-*

   *SEQ . 5*
- Arg-Val-Gly-Phe-Tyr-Glu-Ser-Asp-
   SEQ.34
- Leu-Leu-Ser-Ala-Leu-Val-Glu-Thr-
   SEQ.35
- Glu-Ala-Ile-Pro-Met-Ser-Ile-Pro-
   SEQ.36
- Ile-Ala-Gly-Arg-Ser-Leu-Asn-Pro-
   SEQ.37
- *Ile-Pro-Glu-Asn-Phe-Phe-Gly-Val-*
   *SEQ.10*
- Lys-Pro-Gln-Gln-Phe-Phe-Gly-Leu-
   SEQ.38
- Asp-Val-Ala-Gln-Phe-Val-Leu-Thr-
   SEQ.39
- Asp-Thr-Leu-Glu-Val-Met-Arg-Lys-
   SEQ.40
- Asp-Val-Gly-His-Phe-Arg-Thr-Phe-
   SEQ.41
- Asp-Ser-Gly-Gly-Phe-Met-Leu-Thr-
   SEQ.42
- Arg-Val-Ala-Glu-Met-Arg-Gly-Glu-
   SEQ.43
- Asp-Leu-Gly-Arg-Phe-Gln-Thr-Phe-
   SEQ.44
- Pro-Phe-Ser-Pro-Leu-Val-Ala-Thr-
   SEQ.45
- *Leu-Arg-Ala-Tyr-Leu-Leu-Pro-Ala-*
   *SEQ.13*
- Ala-Pro-Gly-Asn-Ala-Ser-Glu-Ser-
   SEQ.46
- Phe-Ser-Ser-Glu-Ser-Lys-Arg-Glu-
   SEQ.47
- *Arg-Ala-Ile-His-Ile-Gln-Ala-Glu-*
   *SEQ.12*
- *Gly-Pro-His-Leu-Leu-Val-Glu-Ala-*
   *SEQ.28*
- Pro-Pro-Glu-Glu-Leu-Lys-Phe-Gln-
   SEQ.48
- *Gly-Pro-Leu-Gly-Met-Arg-Gly-Leu-*
   *SEQ. 49*
- *Pro-Gln-Gly-Leu-Glu-Ala-Lys-*
   *SEQ. 15*
- Arg-Pro-Lys-Pro-Val-Glu-Nva-Trp-Arg-Glu-Ala-Lys-
   SEQ. 50
- *Pro-Leu-Gly-Leu-Trp-Ala-D-Arg-*
   *SEQ. 16*
- *Pro-Tyr-Ala-Tyr-Trp-Met-Arg-*
   *SEQ. 51*
- *Arg-Pro-Lys-Pro-Leu-Ala-Nva-Trp-*
   *SEQ. 52*
- *Pro-Cha-Gly-Cys(Me)-His-Ala-*
   *SEQ. 20*
- *Pro-Leu-Gly-Leu-Dpa-Ala-Arg-*
   *SEQ. 21*
- *Pro-Leu-Gly-[2-mercapto-4-methyl-pentanoyl]-Leu-Gly-*
   *SEQ. 22*
- *Arg-Pro-Lys-Pro-Tyr-Ala-Nva-Trp-Met-*
   *SEQ. 26*
- *Arg-Pro-Lys-Pro-Val-Glu-Nva-Trp-Arg-*
   *SEQ. 50*

Lorsque la séquence peptidique cible est destinée à être cible de l'enzyme MMP-5, elle peut ainsi correspondre à l'une des séquences suivantes :
*-Arg-Pro-Lys-Pro-Val-Glu-Nva-Trp-Arg-Glu-Ala-Lys-*
   *SEQ. 50*

Lorsque la séquence peptidique cible est destinée à être cible de l'enzyme MMP-7, elle peut ainsi correspondre à l'une des séquences suivantes :
*-Pro-Leu-Gly-Leu-Trp-Ala-D-Arg-*
   *SEQ. 16*
*-Pro-Leu-Gly-Leu-Dpa-Ala-Arg-*
   *SEQ. 21*
*-Arg-Pro-Leu-Ala-Leu-Trp-Arg-Ser-*
   *SEQ. 24*
*-Pro-Cha-Gly-Cys(Me)-His-Ala-*
   *SEQ. 20*
*-Pro-Leu-Gly-[2-mercapto-4-methyl-pentanoyl]-Leu-Gly-*
   *SEQ. 22*
*-Pro-Leu-Ala-Leu-Trp-Ala-Arg-*
   *SEQ. 1*7
*-Pro-Tyr-Ala-Tyr-Trp-Met-Arg-*
   *SEQ. 51*
*-Arg-Pro-Lys-Pro-Leu-Ala-Nva-Trp-*
   *SEQ. 52*

Lorsque la séquence peptidique cible est destinée à être cible de l'enzyme MMP-8, elle peut ainsi correspondre à l'une des séquences suivantes :
*-Pro-Leu-Ala-Tyr-Trp-Ala-Arg-*
   *SEQ. 53*
*-Pro-Leu-Gly-Leu-Trp-Ala-D-Arg-*
   *SEQ. 16*
*-Pro-Cha-Gly-Cys(Me)-His-Ala-*
   *SEQ. 20*
*-Pro-Leu-Gly-Leu-Dpa-Ala-Arg-*
   *SEQ. 21*
*-Pro-Leu-Gly-[2-mercapto-4-methyl-pentanoyl]-Leu-Gly-*
   *SEQ. 22*
*-Pro-Leu-Ala-Leu-Trp-Ala-Arg-*
   *SEQ. 17*
*-Pro-Leu-Gly-Cys(Me)-His-Ala-D-Arg-*
   *SEQ. 23*
*-Arg-Pro-Leu-Ala-Leu-Trp-Arg-*
   *SEQ. 24*
*-Pro-Cha-Gly-Nva-His-Ala-*
   *SEQ. 25*

Lorsque la séquence peptidique cible est destinée à être cible de l'enzyme MMP-9, elle peut ainsi correspondre à l'une des séquences suivantes :
-Gly-Pro-Pro-Gly-Val-Val-Gly-Pro-
   SEQ.54
-Gly-Pro-Pro-Gly-Leu-Arg-Gly-Glu-
   SEQ.55
-Gly-Pro-Gly-Gly-Val-Val-Gly-Pro-
   SEQ.56
-Ile-Pro-Gln-Asn-Phe-Phe-Gly-Val-
   SEQ.57
-Pro-Pro-Gly-Ala-Tyr-His-Gly-Ala-
   SEQ.58
*-Arg-Ala-Ile-His-Ile-Gln-Ala-Glu-*
   *SEQ.12*
*-Gly-Pro-Leu-Gly-Met-Arg-Gly-Leu-*
   *SEQ. 14*
*-Pro-Gln-Gly-Leu-Glu-Ala-Lys-*
   *SEQ. 15*
*-Arg-Pro-Lys-Pro-Val-Glu-Nva-Trp-Arg-Glu-Ala-Lys-*
   *SEQ.50*
*-Pro-Leu-Gly-Leu-Trp-Ala-D-Arg-*
   *SEQ. 16*
*-Pro-Cha-Gly-Cys(Me)-His-Ala-*
   *SEQ. 19*
*-Pro-Leu-Gly-SCH[CH₂CH (CH₃) ₂]-CO-Leu-Gly-*
   *SEQ. 32*
*-Arg-Pro-Pro-Gly-Phe-Ser-Ala-Phe-*
   *SEQ. 33*
*-Pro-Cha-Gly-Cys(Me)-His-Ala-*
   *SEQ. 20*
*-Pro-Leu-Gly-Leu-Dpa-Ala-Arg-*
   *SEQ. 21*
*-Pro-Leu-Gly-[2-mercapto-4-methyl-pentanoyl]-Leu-Gly-*
   *SEQ. 22*
*-Pro-Leu-Gly-Cys(Me)-His-Ala-D-Arg-*
   *SEQ. 23*

Lorsque la séquence peptidique cible est destinée à être cible de l'enzyme MMP-10, elle peut ainsi correspondre à l'une des séquences suivantes :
*-Arg-Ala-Ile-His-Ile-Gln-Ala-Glu-*
   *SEQ.12*
*-Gly-Pro-His-Leu-Leu-Val-Glu-Ala-*
   *SEQ.28*
*-Pro-Leu-Gly-Leu-Dpa-Ala-Arg-*
   *SEQ. 21*

Lorsque la séquence peptidique cible est destinée à être cible de l'enzyme MMP-11, elle peut ainsi correspondre à l'une des séquences suivantes :
*-Pro-Cha-Gly-Cys(Me)-His-Ala-*
   *SEQ. 20*
*-Pro-Leu-Gly-Leu-Dpa-Ala-Arg-*
   *SEQ. 21*

Lorsque la séquence peptidique cible est destinée à être cible de l'enzyme MMP-12, elle peut ainsi correspondre à l'une des séquences suivantes :
*-Pro-Cha-Gly-Cys(Me)-His-Ala-*
   *SEQ. 20*
*-Pro-Leu-Gly-Leu-Dpa-Ala-Arg-*
   *SEQ. 21*
*-Pro-Leu-Gly-[2-mercapto-4-methyl-pentanoyl]-Leu-Gly-*
   *SEQ. 22*
*-Pro-Leu-Ala-Leu-Trp-Ala-Arg-*
   *SEQ. 17*
*-Pro-Leu-Gly-Cys(Me)-His-Ala-D-Arg-*
   *SEQ. 23*
*-Pro-Tyr-Ala-Tyr-Trp-Met-Arg-*
   *SEQ. 51*
*-Arg-Pro-Lys-Pro-Leu-Ala-Nva-Trp-*
   *SEQ. 52*
*-Arg-Pro-Leu-Ala-Leu-Trp-Arg-*
   *SEQ. 24*
*-Pro-Cha-Gly-Nva-His-Ala-*
   *SEQ. 25*
*-Arg-Pro-Lys-Pro-Tyr-Ala-Nva-Trp-Met-*
   *SEQ. 26*
*-Arg-Pro-Lys-Pro-Val-Glu-Nva-Trp-Arg-*
   *SEQ. 50*
*-Arg-Pro-Lys-Pro-Gln-Gln Phe-Trp-*
   *SEQ. 59*

Lorsque la séquence peptidique cible est destinée à être cible de l'enzyme MMP-13, elle peut ainsi correspondre à l'une des séquences suivantes :
*-Gly-Pro-Leu-Gly-Met-Arg-Gly-Leu-*
   *SEQ. 49*
-*Arg-Pro-Lys-Pro-Val-Glu-Nva-Trp-Arg-Glu-Ala-Lys-*
   *SEQ. 50*
-*Pro-Cha-Gly-Nva-His-Ala-Dpa-*
   *SEQ. 25*
-*Pro-Cha-Gly-Cys(Me)-His-Ala-*
   *SEQ. 20*
-*Pro-Leu-Gly-Leu-Dpa-Ala-Arg-*
   *SEQ. 21*
-*Pro-Leu-Gly-[2-mercapto-4-methyl-pentanoyl]-Leu-Gly-*
   *SEQ.22*
*-Pro-Leu-Gly-Leu-Trp-Ala-D-Arg- SEQ. 16*
*-Pro-Leu-Ala-Leu-Trp-Ala-Arg-*
   *SEQ. 17*
*-Pro-Leu-Gly-Cys(Me)-His-Ala-D-Arg-*
   *SEQ. 23*
*-Pro-Leu-Gly-Met-Trp-Ser-Arg-*
   *SEQ. 31*
*-Pro-Tyr-Ala-Tyr-Trp-Met-Arg-*
   *SEQ. 51*
*-Arg-Pro-Lys-Pro-Leu-Ala-Nva-Trp-*
   *SEQ. 52*
*-Arg-Pro-Leu-Ala-Leu-Trp-Arg-*
   *SEQ. 24*
*-Pro-Leu-Ala-Tyr-Trp-Ala-Arg-*
   *SEQ. 53*
*-Pro-Cha-Gly-Nva-His-Ala-*
   *SEQ. 25*
*-Arg-Pro-Lys-Pro-Tyr-Ala-Nva-Trp-Met-*
   *SEQ. 26*
*-Arg-Pro-Lys-Pro-Gln-Gln Phe-Trp-*
   *SEQ. 59*

Lorsque la séquence peptidique cible est destinée à être cible de l'enzyme MMP-14, elle peut ainsi correspondre à l'une des séquences suivantes :
-Pro-Leu-Ala-Cys(p-OMeBz)-Trp-Ala-Arg-
   SEQ. 60
-*Pro-Cha-Gly-Cys(Me)-His-Ala-*
   *SEQ. 20*
-*Pro-Leu-Gly-Leu-Dpa-Ala-Arg-*
   *SEQ. 21*
-*Pro-Leu-Gly-[2-mercapto-4-methyl-pentanoyl]-Leu-Gly-*
   *SEQ. 22*

Lorsque la séquence peptidique cible est destinée à être cible de l'enzyme MMP-15, elle peut ainsi correspondre à l'une des séquences suivantes :
*-Pro-Leu-Gly-Leu-Dpa-Ala-Arg-*
   *SEQ. 21*

Lorsque la séquence peptidique cible est destinée à être cible de l'enzyme MMP-16, elle peut ainsi correspondre à l'une des séquences suivantes :
*-Pro-Leu-Gly-Leu-Dpa-Ala-Arg-*
   *SEQ. 21*

Lorsque la séquence peptidique cible est destinée à être cible de l'enzyme MMP-17, elle peut ainsi correspondre à l'une des séquences suivantes :
*-Pro-Leu-Gly-Leu-Dpa-Ala-Arg-*
   *SEQ. 21*
*-Pro-Leu-Gly-[2-mercapto-4-methyl-pentanoyl]-Leu-Gly-*
   *SEQ. 22*

Lorsque la séquence peptidique cible est destinée à être cible de l'enzyme MMP-19, elle peut ainsi correspondre à l'une des séquences suivantes :
-*Pro-Leu-Gly-Leu-Dpa-Ala-Arg-*
   *SEQ. 21*

Lorsque la séquence peptidique cible est destinée à être cible de l'enzyme MMP-23, elle peut ainsi correspondre à l'une des séquences suivantes :
*-Pro-Leu-Gly-Leu-Dpa-Ala-Arg-*
   *SEQ. 21*

Lorsque la séquence peptidique cible est destinée à être cible de l'enzyme MMP-25, elle peut ainsi correspondre à l'une des séquences suivantes :
*-Pro-Leu-Gly-Leu-Dpa-Ala-Arg-*
   *SEQ. 21*

Lorsque la séquence peptidique cible est destinée à être cible de l'enzyme MMP-26, elle peut ainsi correspondre à l'une des séquences suivantes :
-*Pro-Leu-Gly-Leu-Trp-Ala-D-Arg-*
   *SEQ. 16*

Outre la présence d'une séquence peptidique cible, le composé peut comporter un ou plusieurs groupes liés de façon covalente à ladite séquence peptidique cible, ce ou ces groupes étant aptes à former un complexe avec les groupes formant cage susmentionnés.

Ces groupes peuvent être, des groupes cycliques hydrocarbonés, tels que des groupes aromatiques éventuellement hétérocycliques ou des groupes hydrocarbonés alicycliques.

A titre de groupes aromatiques, éventuellement hétérocycliques, on peut citer des groupes tels que des groupes coumarines, des groupes fluorescéines, des groupes cyanines.

Outre leur capacité à former un complexe « hôte-invité » avec des groupes formant cage, certains de ces groupes peuvent constituer, en plus, des sondes fluorescentes aptes à permettre, par des mesures de variation de fluorescence, la quantification la quantification de l'activité enzymatique.

Des composés adéquats comprenant une séquence peptidique cible de la ou les protéases dont on veut déterminer la présence et/ou l'activité enzymatique peuvent répondre à la formule (VII) suivante :

(X₁)ₙ-S-(X₂)ₘ (VII)

dans laquelle S correspond à une séquence peptidique cible de la protéase (dont on veut déterminer la présence et/ou l'activité enzymatique), cette séquence pouvant correspondre à l'une de celles explicitées ci-dessus pour les enzymes MMP ;
- n et m sont des entiers égaux à 0 ou 1, étant entendu que l'un au moins des n et m est différent de zéro ;
- lorsque m et n sont égaux à 1, X₁ est une sonde porteuse d'un groupe donneur fluorescent et X₂ est une sonde porteuse d'un groupe accepteur fluorescent ou non fluorescent, le groupe donneur et le groupe accepteur étant choisis, de préférence, de sorte à ce que le spectre d'émission de fluorescence du groupe donneur recouvre, au moins en partie, le spectre d'absorption du groupe accepteur, sachant que l'un au moins des X₁ ou X₂ comporte un groupe apte à se complexer avec un groupe formant cage susmentionné.

Dans ce qui précède et ce qui suit, par groupe donneur fluorescent, on entend classiquement un groupe apte à absorber de l'énergie lumineuse (dite lumière d'excitation) et de la restituer sous deux formes possibles en fonction de son environnement :
a) sous forme d'un transfert d'énergie d'excitation par résonance avec un groupe accepteur, à condition que ce dernier se trouve à une distance compatible avec le phénomène de FRET décrit précédemment et préférentiellement, à une distance allant de 10 à 100 Å ; ou
b) sous forme d'une lumière fluorescente (dite lumière d'émission du donneur), dont la longueur d'onde appartient, généralement, au domaine du visible.

Par groupe accepteur, on entend classiquement, au sens de l'invention, un groupe apte à absorber l'énergie d'excitation du groupe donneur par énergie de résonance. Cette énergie d'excitation pourra être restituée selon deux grands mondes :
a) par émission radiative (émission de photons), auquel cas on parlera de groupe accepteur fluorescent ; ou
b) par émission non radiative (à savoir tout autre mode de désactivation autre que l'émission de photons), auquel cas on parlera de groupe accepteur non fluorescent.

De préférence, X₁ est une sonde porteuse d'un groupe donneur fluorescent et X₂ est une sonde porteuse d'un groupe accepteur fluorescent.

Par sonde, on entend classiquement, au sens de l'invention, un reste d'acide aminé porteur d'un groupe donneur (dans le cas de la sonde X₁) ou porteur d'un groupe accepteur (dans le cas de la sonde X₂).

Ainsi, en présence d'une protéase donnée, dont la séquence S est une séquence cible de cette enzyme, le substrat va se cliver au niveau de la séquence S, ce qui va générer un éloignement dans l'espace des sondes X₁ et X₂. S'ensuit un éloignement des groupes donneur et accepteur et, par voie de conséquence, une variation du transfert de l'énergie de résonance entre ces deux groupes. A partir de la variation d'énergie de résonance, il est ainsi possible de remonter à l'activité enzymatique de l'enzyme MMP susmentionnée.

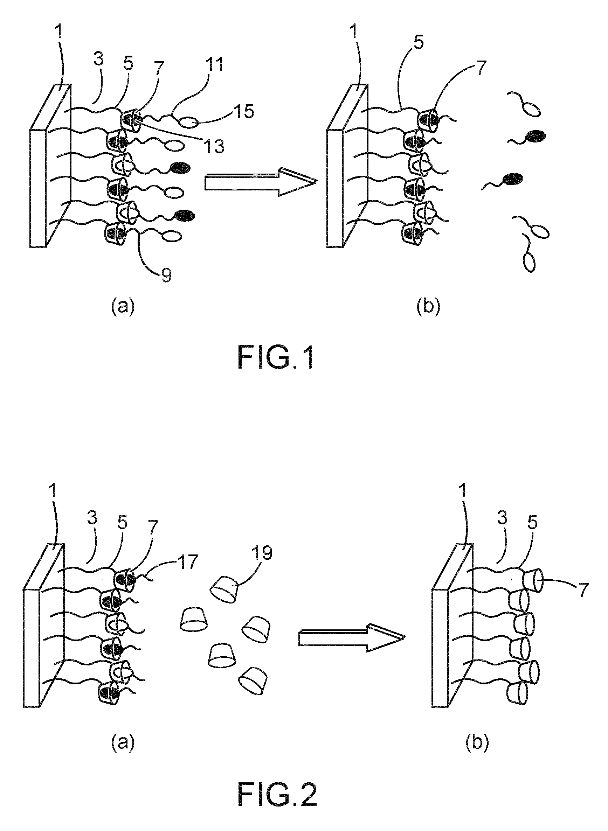

Ce principe est illustré sur la figure 1 qui représente :
- sur la partie (a), un biocapteur comprenant un support 1 fonctionnalisé par des composés 3 comprenant un bras espaceur 5 lié de façon covalente au support 1 et un groupe d'extrémité du type β-cyclodextrine 7 (symbolisé par un cône tronqué), chaque groupe β-cyclodextrine étant complexé à un composé 9 comportant une séquence peptidique cible 11 et deux sondes 13, 15 placées aux extrémités de la séquence peptidique cible ;
- sur la partie (b), les mêmes éléments que sur la partie (a), si ce n'est que chaque séquence peptidique a été clivée suite à la mise en contact du biocapteur avec une protéase apte à cliver ladite séquence peptidique cible, moyennant quoi il y a éloignement des sondes de chacun des composés susmentionnés.

Comme mentionné ci-dessus, les composés comprenant une séquence peptidique cible de la ou les protéases, dont on veut déterminer la présence et/ou l'activité protéasique, peuvent comprendre au moins un sonde X₁ porteuse d'un groupe donneur fluorescent et au moins une sonde X₂ porteur d'un groupe accepteur fluorescent ou non fluorescent, de sorte à ce que, de préférence, le spectre d'émission de fluorescence du groupe donneur recouvre, au moins en partie, le spectre d'absorption du groupe accepteur, ce qui se traduit par un phénomène de transfert non radiatif de l'énergie d'excitation du groupe donneur vers le groupe accepteur (ou transfert d'énergie de résonance).

Les composés présentant de telles sondes vont permettre de suivre l'activité enzymatique d'une protéase donnée (dès lors que la séquence peptidique cible dudit composé est cible de ladite enzyme) :
- par variation du transfert d'énergie par résonance, grâce, par exemple, à un microscope d'épifluorescence ; et
- par augmentation de la fluorescence de l'une des sondes fluorescentes après clivage de la séquence peptidique cible.

De tels groupes (donneur fluorescent et accepteur fluorescent ou non fluorescent) comprennent classiquement un noyau aromatique, tel qu'un noyau benzénique, anthracénique ou coumarine.

On peut citer, par exemple, les couples suivants (le premier membre du couple étant le groupe donneur, tandis que le second membre du couple étant le groupe accepteur) :
*Tryptophane/2,4-dinitrophényle (symbolisé par l'abréviation W/Dnp) ;
*Acide o-aminobenzoïque/2,4-dinitrophényle (symbolisé par l'abréviation Abz/Dnp) ;
*(7-méthoxycoumarin-4-yl)-acétyle/2,4-dinitrophényle (symbolisé par l'abréviation Mca/Dnp) ;
*(7-méthoxycoumarin-4-yl)-acétyle/N-3-(2,4-dinitrophényl)-L-2,3-diaminopropyle (symbolisé par l'abréviation Mca/Dpa) ;
*Tryptophane/Dansyle (symbolisé par l'abréviation W/Dns) ;
*N-méthylanthranoyle/2,4-dinitrophényle (symbolisé par l'abréviation Nma/Dnp) ;
*6,7-diméthoxycoumarin-4-yl-acétyle/acide 6-N-(7-nitrobenz-2-oxa-1,3-diazol-4-yl)aminohexanoïque (symbolisé par l'abréviation DMC/Nbd) ;
*Acide 5-(2'-aminométhyl)naphtalène sulfonique/acide 4-(4'-diméthylaminophénylaza)benzoïque (symbolisé par l'abréviation EDANS/Dabcyl) ;
* acide 7-méthoxycoumarin-3-carboxylique/acide 7-diéthylaminocoumarin-3-carboxylique/ (symbolisé par l'abréviation MC/DAC), lesdits groupes répondant aux formules suivantes :

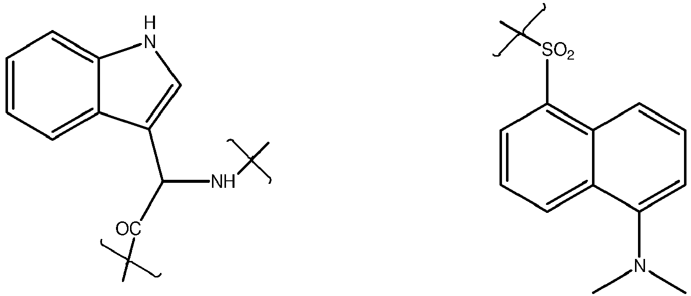

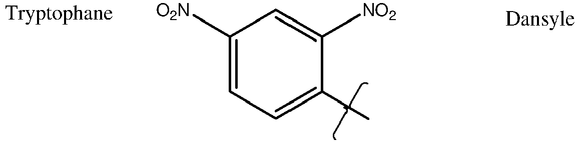

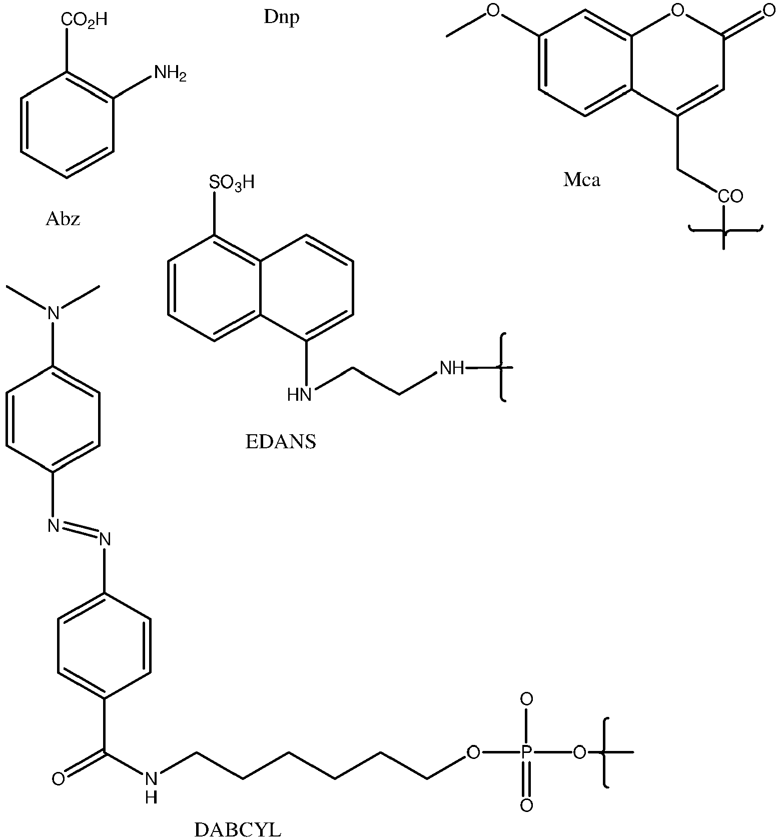

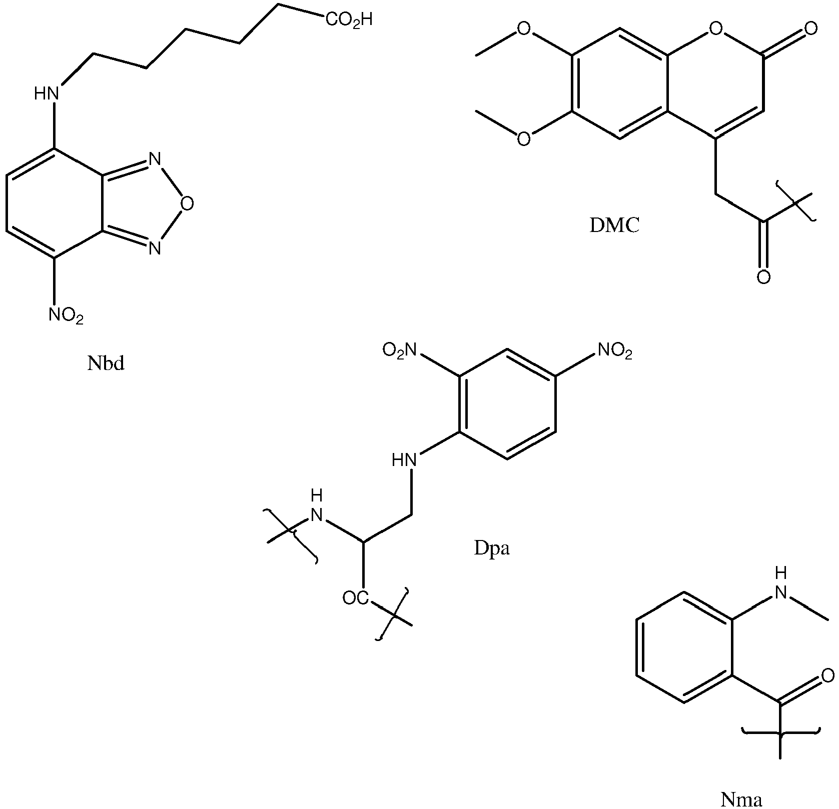

Avantageusement, le groupe donneur et le groupe accepteur sont des groupes comprenant un noyau coumarine.

Du choix d'utiliser des groupes comprenant un noyau coumarine pour constituer les groupes donneur et accepteur découlent les avantages suivants :
- ils présentent des longueurs d'onde d'absorption et d'émission de fluorescence en dehors de celles des séquences peptidiques, ce qui permet d'éviter des phénomènes de bruit de fond lors de la mesure de l'activité enzymatique ;
- ils permettent d'obtenir un recouvrement spectral maximal entre l'émission de fluorescence du groupe donneur et l'absorption de fluorescence du groupe accepteur ;
- ils présentent un bon rendement quantique et une stabilité au photoblanchiment ;
- ils présentent une taille telle qui n'engendre pas de perturbation de la reconnaissance enzymatique de la séquence cible par l'enzyme MMP dont on veut mesurer l'activité ;
- ils présentent une excellente capacité intrinsèque à se lier avec un groupe formant cage, tel qu'un groupe β-cyclodextrine pour former un complexe « hôte-invité ».

En particulier, le couple préféré peut être le couple MC/DAC, ayant pour formules respectives, une fois liés à la sonde, les formules suivantes :

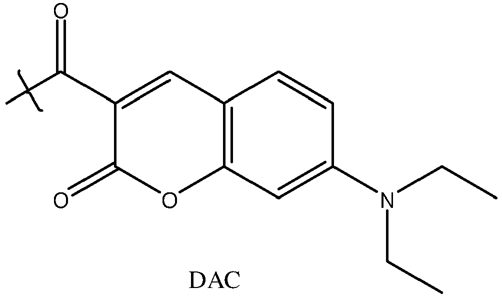

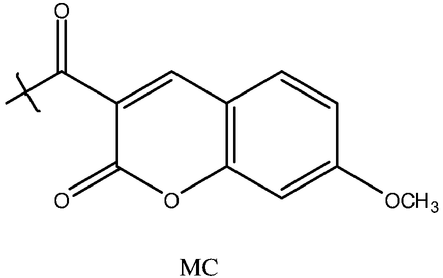

MC étant un groupe donneur fluorescent pouvant être porté par X₁ et DAC étant un groupe accepteur pouvant être porté par X₂.

Les sondes X₁ et X₂ peuvent être des restes d'acide aminé.

Un exemple de sonde X₁ peut être ainsi la sonde -Lys(MC) de formule suivante : 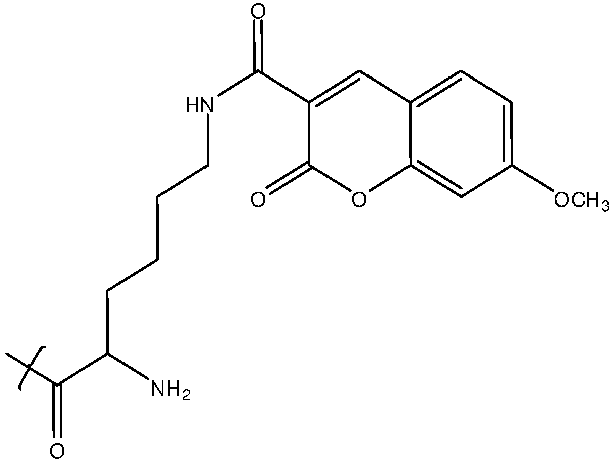 le groupe -NH₂ libre pouvant être engagé dans une liaison amide avec un autre acide aminé ;
tandis qu'un exemple de sonde X₂ peut être la sonde -Lys(DAC) de formule suivante :

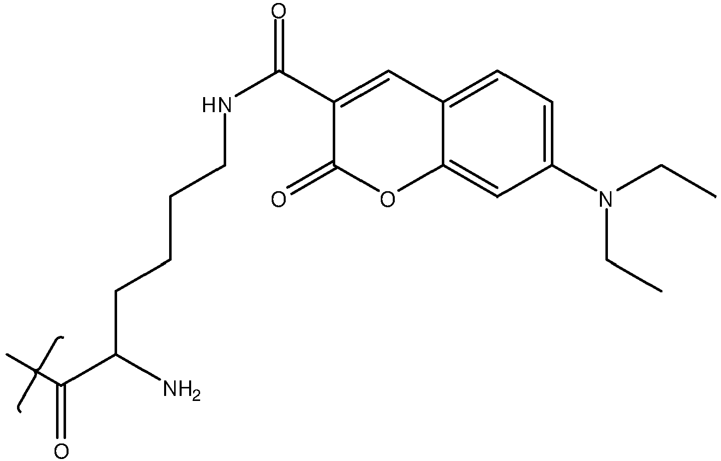

Ce couple de sondes peut être avantageusement utilisé, lorsque les groupes formant cage sont des groupes β-cyclodextrines, ces groupes β-cyclodextrines formant un complexe hôte-invité avec de telles sondes, grâce à la présence de groupes coumarines.

Les groupes hydrocarbonés cycliques aptes à former un complexe avec les groupes formant cage peuvent être également des groupes adamantanes, ces groupes pouvant être associés, au sein du composé comprenant une séquence peptidique cible, à des sondes fluorescentes telles que mentionnées ci-dessus.

Des exemples de composés comprenant une séquence peptidique cible peuvent répondre à la formule (VII) suivante :

A-NH-D(CH₂-C(=O)-R₂-B)₂ (VII)

dans laquelle :
A est un groupe comprenant une séquence peptidique cible de la protéase dont on veut déterminer la présence et/ou l'activité enzymatique;
B est un groupe adamantane;
D représente un atome d'azote ; et
R₂ représente une liaison simple ou un groupement de liaison comprenant de 1 à 40 atomes de carbone et notamment de 1 à 30 atomes de carbone.

Le groupe R₂ peut être un groupe (hétéro)alkylène, éventuellement substitué. Dans le cadre de la présente invention, on entend, par « groupe alkylène », un groupe hydrocarboné saturé, linéaire, ramifié ou cyclique, pouvant comprendre de 1 à 30 atomes de carbone, notamment de 1 à 20 atomes de carbone et, en particulier, de 1 à 10 atomes de carbone.

Dans le cadre de la présente invention, on entend, par « groupe hétéroalkylène », un groupe hydrocarboné saturé, linéaire, ramifié ou cyclique, comprenant au moins un hétéroatome et pouvant comprendre de 1 à 30 atomes de carbone, notamment de 1 à 20 atomes de carbone et, en particulier, de 1 à 10 atomes de carbone, le ou les hétéroatomes pouvant être N, O, P, Si ou S et notamment N, O, ou S. Les hétéroatomes peuvent en particulier interrompre une chaîne alkylène, démarrer une chaîne alkylène et/ou terminer une chaîne alkylène.

En particulier, R₂ peut être un groupe héteralkylène pouvant comprendre de 1 à 30 atomes et comportant un ou plusieurs atomes d'oxygène, le ou les atomes d'oxygène pouvant interrompre une chaîne alkylène, démarrer une chaîne alkylène et/ou terminer une chaîne alkylène, un groupe R₂ spécifique répondant à cette définition pouvant être un groupe -(OC₂H₄)₄-O-.

Concernant le groupe A susmentionné, celui-ci peut comprendre, outre une séquence peptidique cible de la protéase susmentionnée, une ou plusieurs sondes, telles que, par exemple, celles mentionnées ci-dessus.

Des composés spécifiques à l'invention conformes à la définition donnée ci-dessus sont des composés répondant à la formule (VIII) suivante :

A₁-NH-N(CH₂-C(=O)-(C₂H₄O)₄-O-B)₂ (VIII)

dans laquelle :
- A₁ est un groupe comprenant une séquence peptidique cible d'une protéase (telle qu'une enzyme MMP, la séquence peptidique cible pouvant être ainsi l'une de celles listées ci-dessus) et comprenant au moins une sonde telle que mentionnée ci-dessus ;
- B est un groupe adamantane.

Plus spécifiquement, A₁ peut correspondre à l'une des formules suivantes :
Lys(DAC)-Gly-Pro-Gln-Gly-Leu-Leu-Gly-Ala-Lys(MC)-Ala-CO-
Lys(DAC)-Pro-Pro-Gly-Ala-Tyr-His-Gly-Ala-Lys(MC)-Ala-CO-
Lys(DAC)-Gly-Pro-Gly-Gly-Val-Val-Gly-Pro-Lys(MC)-Ala-CO-
Lys (DAC) et Lys(MC) répondant aux mêmes définitions que celles données ci-dessus, le groupement -CO- est un groupe issu du groupe -COOH appartenant au reste alanine après réaction avec un groupe amine et les séquences peptidiques situées entre le groupement Lys(DAC) et le groupement Lys(MC) correspondant respectivement à des séquences peptidiques cibles des enzymes MMP-1, MMP-2 et MMP-9.

De par l'utilisation d'un groupe formant cage, tel qu'un groupe β-cyclodextrine, formant un complexe avec au moins un des groupes portés par le ou les composés comprenant une séquence peptidique cible de la ou les protéases dont on veut déterminer la présence et/ou l'activité enzymatique, complexe dont l'interaction entre l'hôte-invité n'est pas de nature covalente, il ressort ainsi qu'il est possible de régénérer le biocapteur, c'est-à-dire de dissocier le complexe « hôte-invité » de sorte à pouvoir rendre le biocapteur réutilisable, en mettant en contact tout simplement le biocapteur utilisé avec une solution comprenant des composés comprenant au moins un groupe formant cage tel que défini ci-dessus, lesquels composés étant présents, dans la solution, à un concentration appropriée, de sorte de permettre de déplacer, par formation d'un complexe « hôte-invité », les restes de séquences cibles fixés au biocapteur via les groupes formant cage compris dans la solution, moyennant quoi, après lavage, l'on récupère un biocapteur « neuf ».

En d'autres termes, la mise en contact d'un composé comprenant au moins un groupe formant cage avec le biocapteur utilisé va engendrer, par une réaction de dissociation par compétition, du complexe « hôte-invité » laissant un biocapteur comprenant un support fonctionnalisé par les composés comprenant un groupe formant cage initiaux libres d'accueillir de nouveaux composés comprenant une séquence peptidique cible pour une nouvelle opération de détermination de la présence d'une protéase et/ou de la quantification de l'activité enzymatique de celle-ci.

Ce principe est illustré sur la figure 2, qui représente :
- sur la partie (a), un biocapteur comprenant un support 1 fonctionnalisé par des composés 3 comprenant un bras espaceur 5 lié de façon covalente au support 1 et un groupe d'extrémité du type groupe formant cage 7 (symbolisé par un cône tronqué), chaque groupe formant cage étant complexé à un reste de composé comprenant une séquence peptidique cible 17 et des composés libres en solution comprenant un groupe formant cage 19 ;
- sur la partie (b), les mêmes éléments que sur la partie (a), si ce n'est que les groupes formant cage liés au support via un bras espaceur sont libres, de sorte à pouvoir accueillir de nouveaux composés comprenant une séquence peptidique libre en vue d'une nouvelle utilisation du biocapteur.

Du fait du caractère régénérable des biocapteurs de l'invention, il est ainsi possible d'envisager leur utilisation pour effectuer différentes mesures d'activité enzymatique de manière séquentielle (à savoir, la mise en oeuvre d'au moins un cycle d'étapes comprenant la mise en contact d'un support inactif avec un substrat d'une protéase dont on veut déterminer l'activité suivie du test enzymatique approprié, la régénération du support puis une mise en contact dudit support ainsi régénéré avec un substrat d'une autre protéase dont on veut déterminer l'activité suivie du test enzymatique approprié).

Les biocapteurs de l'invention peuvent être intégrés dans des systèmes comprenant un dispositif permettant d'émettre une source lumineuse excitatrice (un tel système pouvant être une diode électroluminescente organique (connue sous l'abréviation de diode OLED) et un dispositif de mesure de variation de fluorescence (tel qu'un spectrofluorimètre ou un microscope d'épifluorescence).

Ainsi, le signal émis par le biocapteur pourra être suivi des façons suivantes :
- par augmentation de l'intensité de fluorescence des sondes telles que définies ci-dessus, se trouvant sur la partie peptidique relarguée après hydrolyse du composé comprenant une séquence peptidique libre ;
- par variation de l'énergie de résonance (FRET) sous réserve que le composé comprenant une séquence peptidique libre comprenne des sondes telles que définies ci-dessus.

Les biocapteurs de l'invention sont destinés à être utilisés pour déterminer la présence de protéase(s), telles que des enzymes MMP, voire pour quantifier l'activité enzymatique de ces dernières. De ce fait, ils peuvent être destinés à être mis en contact avec des fluides biologiques, dont on veut savoir s'il contient certaines protéases et, le cas échéant, mesurer l'activité enzymatique de ces enzymes ou avec des milieux comprenant des cultures cellulaires ou cellules tumorales et la protéase, dont on veut déterminer l'activité de la protéase.

Les biocapteurs de l'invention peuvent être préparés par un procédé comprenant les étapes suivantes :
*une étape de fonctionnalisation d'un support, moyennant quoi l'on obtient un support fonctionnalisé par des composés organiques comprenant au moins un groupe formant cage, tel qu'un groupe β-cyclodextrine ;
*une étape de mise en contact du support ainsi fonctionnalisé avec au moins un composé comprenant une séquence peptidique cible de la ou les protéases dont on veut déterminer la présence et/ou l'activité enzymatique.

L'étape de fonctionnalisation peut être réalisée selon de nombreuses variantes.

L'étape de fonctionnalisation, selon une première variante, peut être réalisée par la succession d'opérations suivantes :
- éventuellement, une opération d'activation de la surface d'un support solide, de façon à créer des fonctions aptes à réagir avec un groupe d'un premier composé pour former une liaison covalente ;
- une opération de mise en contact dudit support avec ledit premier composé, lequel premier composé comprend un groupe apte à réagir avec le support pour être lié à ce dernier (par exemple, par le biais d'une liaison covalente), éventuellement un groupe espaceur et un groupe terminal, ledit groupe terminal étant apte à réagir avec un groupe d'un composé comprenant un groupe formant cage, ce groupe terminal pouvant être sous une forme protégée, moyennant quoi l'on obtient un support fonctionnalisé par ledit premier composé ;
- après une éventuelle opération de déprotection du groupe terminal protégé, une opération de mise en contact du support ainsi traité avec ledit composé comprenant un groupe formant cage, moyennant quoi le groupe terminal susmentionné réagit avec un groupe du composé comprenant un groupe formant cage pour former une liaison covalente.

Ainsi, le procédé de l'invention peut comprendre une étape d'activation du support pour créer des fonctions réactives.

Classiquement, lorsque le support est un support en silicium, l'étape d'activation peut consister à soumettre le support à un traitement d'oxydation de sorte à créer des groupes -OH à la surface du support, ce traitement d'oxydation pouvant consister à soumettre le support à un flux d'ozone pendant une durée appropriée et également à nettoyer la surface du support de toute pollution organique (laquelle pourrait constituer, par exemple, une couche barrière entre le support en tant que tel que la couche de composés organiques fonctionnalisant ce support).

Selon la première variante susmentionnée, le support peut être un support en silicium, tandis que le premier composé peut répondre à l'une des formules suivantes: 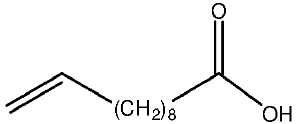 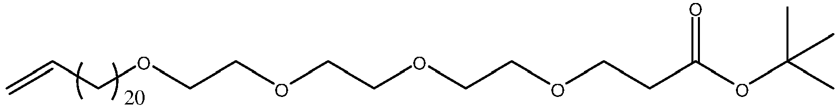 tandis que le composé comprenant un groupe formant cage peut, lorsque ce groupe formant cage, est un groupe β-cyclodextrine, répondre à la formule suivante :

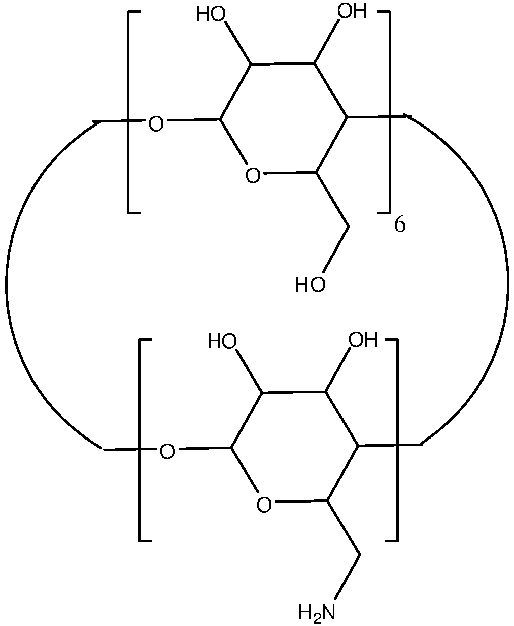

Il est à noter que le premier composé du type susmentionné peut être soit disponible commercialement, soit synthétisé préalablement par des techniques classiques de synthèse organique.

L'étape de fonctionnalisation, selon une deuxième variante, peut être réalisée par la succession d'opérations suivantes :
- éventuellement, une opération d'activation de la surface d'un support solide, de façon à créer des fonctions aptes à réagir avec un groupe d'un premier composé pour former une liaison covalente ;
- une opération de mise en contact dudit support avec ledit premier composé, lequel premier composé comprend un groupe apte à réagir avec le support pour être lié à ce dernier (par exemple, par le biais d'une liaison covalente), éventuellement un groupe espaceur et un groupe terminal consistant en un groupe formant cage, moyennant quoi l'on obtient un support fonctionnalisé par ledit premier composé.

Un exemple de premier composé conforme à la deuxième variante peut être un composé de formule suivante : 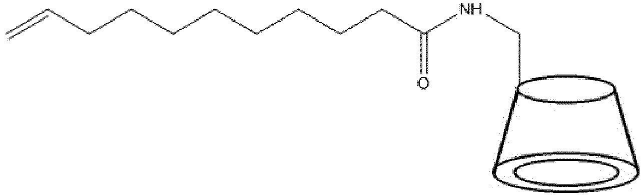 le symbole en forme de cône tronqué correspondant au groupe de formule suivante : 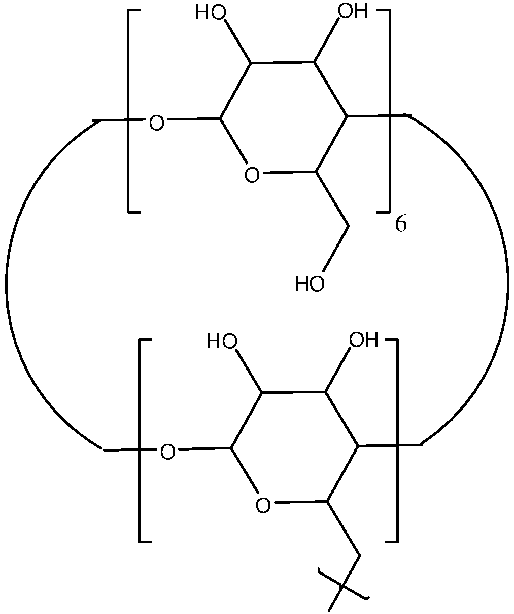 l'accolade indiquant l'endroit par lequel est lié ce groupe au groupement -CH₂-NH- du premier composé.

L'étape de fonctionnalisation, selon une troisième variante, peut être réalisée
- une opération de mise en contact du support avec une solution comprenant un sel de diazonium (constituant un groupe initiateur de polymérisation) et un monomère comportant un groupe pendant apte à réagir avec un composé comprenant un groupe formant cage, moyennant quoi l'on obtient une croissance de chaîne polymérique, par polymérisation dudit monomère, à partir du groupe diazonium qui s'est fixé à la surface du support ;
- une opération de mise en contact dudit support ainsi obtenu avec ledit composé comprenant un groupe formant cage, moyennant quoi ledit composé réagit avec le groupe pendant présent sur les chaînes polymériques pour former une liaison covalente.

Le support ainsi fonctionnalisé, quelle que soit la variante de réalisation, est ensuite mis en contact avec un composé comprenant une séquence peptidique cible d'une protéase, dont on veut déterminer la présence et/ou l'activité enzymatique, ce composé comprenant un groupe apte à se complexer selon un complexe hôte-invité avec le groupe formant cage.

L'invention va, à présent, être décrite en référence aux exemples donnés ci-dessous à titre illustratif et non limitatif.

### BRÈVE DESCRIPTION DES DESSINS

La figure 1 représente un schéma illustrant le principe de détection des protéases mettant en oeuvre un biocapteur conforme à l'invention.
La figure 2 représente un schéma illustrant le principe de régénération des biocapteurs conformes à l'invention.

### EXPOSÉ DÉTAILLÉ DE MODES DE RÉALISATION PARTICULIERS

Les exemples présentés ci-dessous illustrent la préparation de supports fonctionnalisés destinés à entrer dans la constitution de biocapteurs conformes à l'invention.

### Exemple 1

Cet exemple illustre la préparation d'un support fonctionnalisé conforme à l'invention pouvant être représenté par le schéma suivant : 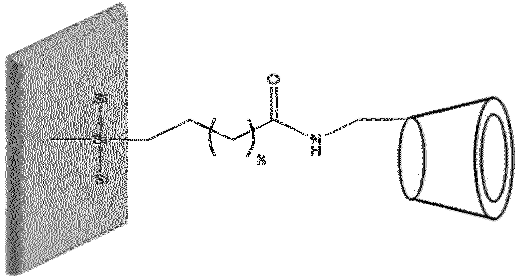 ce support consistant en un support en silicium lié à des groupes β-monoaminocyclodextrines via un groupe espaceur de formule -(CH₂)₁₀-CO-, ce groupe espaceur étant lié aux groupes β-monoaminocyclodextrines par l'unique groupe -NH-CH₂- appartenant à ce groupe β-monoaminocyclodextrine.

La préparation de ce support comprend les étapes suivantes :
- une étape de synthèse du composé N-mono-6N-β-cyclodextrine-10-undéc-10-énamide (étape b) impliquant la préparation préalable du composé intermédiaire 6-monoamino-β-cyclodextrine (étape a) ;
- une étape de couplage du composé préparé à l'étape b avec le support en silicium (étape c), moyennant quoi l'on obtient le support fonctionnalisé représenté schématiquement ci-dessus.

### Etape a-Préparation de la 6-monoamino-β-cyclodextrine

Cette étape a) a trait à la préparation de la 6-monoamino-β-cyclodextrine de formule suivante : 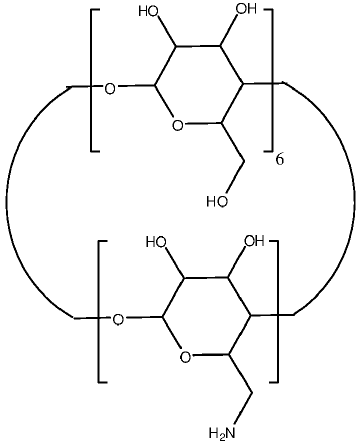 la préparation de ce composé impliquant la préparation préalable de la 6-monoazido-β-cyclodextrine (le NH₂ de la formule ci-dessus étant remplacée dans ce cas par un groupe azido -N₃) et de la 1-mono-6-O-p-tolysulfonylcyclomaltoheptaose (le NH₂ de la formule ci-dessus étant remplacée dans ce cas par un groupe -O-tosylate)

### Etape a1-Préparation de la 1-mono-6-O-p-tolysulfonylcyclomaltoheptaose

Dans un tricol de 1L, on introduit 65 g (0,95 mol) d'imidazole et 250 mL de dichlorométhane. On mélange le tout sous forte agitation à température ambiante, jusqu'à dissolution complète des réactifs puis on refroidit l'ensemble jusqu'à 0°C à l'aide d'un bain de glace tout en maintenant l'agitation. On dissout ensuite 80 g de chlorure de tosyle dans 250 mL de dichlorométhane, l'ensemble étant ensuite ajouté dans le tricol par l'intermédiaire d'une ampoule de coulée au goutte-à-goutte pendant 1H30. Il apparaît un précipité blanc. Après toute une nuit à 4°C, on agite vigoureusement le mélange réactionnel à température ambiante pendant 2 heures. Le précipité est ensuite filtré sur fritté n°3 recouvert de silice et de célite suivi d'un lavage avec un mélange acétate d'éthyle/hexane (1 :1). On transvase le filtrat dans un ballon et on évapore le solvant sous pression réduite. Après ajout de 50 mL d'acétate d'éthyle et 500 mL d'hexane, on filtre sur fritté n°3, le solide blanc obtenu étant séché sous vide dans un dessicateur, ce solide blanc étant le tosylimidazole.

Dans un tricol de 2L équipé d'un thermomètre, d'un réfrigérant et d'une ampoule de coulée, on dissout 40 g de β-cyclodextrine (35,2 mmol) dans 900 mL d'eau. On agite le tout et on chauffe à 60°C. Lorsque la solution devient limpide, on arrête le chauffage et on laisse revenir le mélange à température ambiante. On ajoute en une fois 31,3 g (141 mmol) de tosylimidazole préparé préalablement (le solide blanc mentionné ci-dessus) le plus finement divisé possible et on agite pendant deux heures. Par le biais de l'ampoule de coulée, on ajoute au goutte-à-goutte une solution de soude NaOH (18 g de NaOH dans 50 mL d'eau) et on agite 10 minutes. On sépare ensuite le tosylimidazole, qui n'a pas réagi, par filtration sur fritté n°3. On ajoute au filtrat 48,2 g de NH₄Cl pour stopper la réaction et on agite vigoureusement pour avoir une dissolution complète. On fait passer ensuite un flux d'air à la surface de la solution pendant près de 24 heures. On obtient un large précipité que l'on filtre sur fritté n°3 et on lave deux fois avec 100 mL d'eau glacée puis une fois avec 200 mL d'acétone. Le solide blanc obtenu est alors séché sous vide dans un dessicateur. On récupère environ 18,3 g de produit.
Les résultats de RMN ¹H sont les suivants.
RMN ¹H (DMSO-d⁶) δ(ppm) : 7,43 (d, 2H) ; 7,24 (d, 2H) ; 5,70 (m, 14H) ; 4,80 (m, 7H) ; 4,45 (m, 6H) ; 4,29 (dd, 1H) ; 3,60 (m, 28H) ; 2,41 (s, 3H).

### Etape a2-Préparation de la 6-monoazido-β-cyclodextrine

Dans un ballon de 50 mL, on introduit 2 g de 1-mono-6-O-p-tolysulfonylcyclomaltoheptaose préparé préalablement (1,57 mmol) et 2,04 g d'azoture de sodium NaN₃, que l'on dissout dans 20 mL de diméthylformamide (DMF). Sous flux d'argon, on agite ledit ballon et on le chauffe dans un bain d'huile à environ 80°C pendant 20 heures. On arrête ensuite le chauffage et on maintient l'agitation jusqu'au retour à température ambiante. On verse ensuite le contenu du ballon dans un Erlenmeyer contenant 1 litre d'acétone et on obtient immédiatement un précipité blanc. Ce dernier est filtré sous fritté n°3 et mis à sécher sous vide dans un dessicateur. On récupère alors 3,63 g de produit. Ce solide est ensuite purifié par chromatographie liquide à haute pression (CHLP) en phase inverse.
Les résultats de RMN ¹H sont les suivants.
RMN ¹H (DMSO-d⁶) δ(ppm) : 5,70 (m, 13H) ; 5,60 (d, 1H) ; 4, 87 (d, 1H) ; 4, 80 (m, 6H) ; 4, 45 (m, 6H) ; 3,60 (m, 6H).

### Etape a3-Préparation de la 6-monoamino-β-cyclodextrine

Dans un ballon de 100 mL, on introduit 1 g (0,86 mmol) de 6-monoazido-β-cyclodextrine préparée préalablement, que l'on dissout dans 200 mL de diméthylformamide (DMF) sous agitation. On ajoute ensuite 0,5 g de triphénylphosphine PPh₃ (1,9 mmol) et on chauffe le ballon dans un bain d'huile à 90°C pendant 18 heures. On laisse ensuite revenir à température ambiante la solution présente dans le ballon, qui a pris une couleur jaune. On introduit la solution dans 1 litre d'éthanol chaud avant de filtrer l'ensemble sur fritté chaud. Le solide est ensuite redissous dans un minimum d'eau (environ 10 mL) puis congelé dans de l'azote liquide pour être lyophilisé.
Les résultats de RMN ¹H sont les suivants.
RMN ¹H (DMSO-d⁶) δ(ppm) : 5,75 (m, 14H) ; 4,89 (d, 1H) ; 4,80 (m, 6H) ; 3,60 (m, 26H) ; 3,03 (m, 1H) ; 2,80 (m, 1H).

### Etape b- Préparation du N-mono-6N-β-cyclodextrine-10-undéc-10-énamide

Cette étape b) a trait à la préparation de la N-mono-6N-β-cyclodextrine-10-undéc-10-énamide de formule suivants : 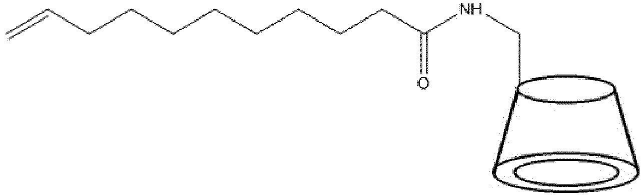 Le protocole précis de préparation de ce composé est le suivant.
92,14 mg (0,5 mmol) d'acide undécènoïque sont dissous dans 4 mL de diméthylformamide (DMF) anhydre sous atmosphère inerte et l'ensemble est placé à 4°C. 46,9 µL de diisopropylcarbodiimide sont ajoutés à la solution. Après agitation pendant 2 heures à température ambiante, une solution de 6-monoamino-β-cyclodextrine (340 mg, 0,3 mmol dans 4 mL de diméthylformamide) préparée préalablement est ajoutée. Après 24 heures, l'ensemble est introduit dans 60 mL d'acétone, moyennant quoi il apparaît un précipité gris. Le solide grisâtre est filtré sur fritté de type 4 puis rincé 4 fois avec de l'acétone. Le précipité est ensuite séché sous vide poussé pendant 12 heures. On récupère 259,9 mg (0,2 mmol) du composé, soit un rendement de 65%.
Les résultats de RMN ¹H sont les suivants.
RMN ¹H (DMSO-d⁶) δ (ppm) : 7,95 (m, 1H) ; 5,75 (m,1H) ; 5,0 (m, 2H) ; 5,78-5,63 (m, 14H) ; 4,90-4,85 (m, 7H) ; 4,50-4,45 (m, 6H) ; 3,66-3,54 (m, 28H) ; 3,42-3,24 (m, 16H) ; 2,15 (m, 4H) ; 1,31-1,22 (m, 10H).

### Etape c-Couplage du composé préparé à l'étape b avec un substrat en silicium

6,2 mg (4,77*10⁻⁶ mol) de N-mono-6N-β-cyclodextrine-10-undéc-10-énamide préparé à l'étape b susmentionnée sont pesés et introduits dans un réacteur du type « ACE pressure tube ». L'ensemble est placé dans une boîte à gants sous atmosphère inerte (à savoir, de l'argon comprenant 10 ppm de O₂ et 2 ppm d'eau).

Du mésitylène est distillé dans la boîte à gants. La teneur en eau (2 ppm) du mésitylène est déterminée par la méthode coulométrique de Karl-Fischer. Quatre morceaux de silicium préalablement traités sont introduits dans le réacteur susmentionné. Le réacteur est ensuite scellé sous argon. L'ensemble est enfin sorti de la boîte à gants et est placé à 140°C dans un bain d'huile pendant 24 heures. Les morceaux de silicium ainsi obtenus sont ensuite rincés sous éthanol, sous sonication pendant 10 minutes et séchés sous azote.

Comme mentionné ci-dessus, les morceaux de silicium avant leur introduction dans le réacteur sont traités préalablement selon le protocole exposé ci-dessous.

Des morceaux (1 cm²) de silicium (111, 18 mΩ, poli sur 2 faces) sont découpés à l'aide d'une pointe en diamant, nettoyés dans un bain à ultrasons en présence successivement de chloroforme, d'acétone et d'éthanol puis séchés sous flux d'azote.

Afin d'obtenir une surface complètement hydrogénée, la couche d'oxyde (SiO₂) doit être enlevée.

Pour ce faire, les morceaux de silicium subissent le protocole suivant :
a) ils sont plongés quelques minutes dans une solution piranha (H₂SO₄/H₂O₂ : 1/1, 60°C) . Cette première étape a pour but de détruire toute contamination organique à la surface des morceaux de silicium ; puis
b) ils sont plongés quelques minutes dans une solution de HF/NH₄F (1/1). L'acide fluorhydrique a pour action d'éliminer la couche d'oxyde de silicium. En raison de la structure cristalline de type 111 du silicium, l'acide fluorhydrique élimine le dioxyde de silicium en formant des pyramides à la surface des morceaux de silicium. NH₄F permet de niveler la surface des morceaux de silicium en détruisant ces pyramides ; puis
c) ils sont plongés de nouveau quelques minutes dans une solution piranha (H₂SO₄/H₂O₂ : 1/1, 60°C) ; puis
d) ils sont soumis à une solution de NH₄F (45%) préalablement dégazée à l'argon.

Après ces étapes, les morceaux de silicium sont placés dans un tube de Schlenk sous vide poussé avant leur transfert en boîte à gants (BAG). Ce protocole permet d'obtenir des morceaux de silicium complètement hydrogénée et relativement stables à l'oxydation dans le temps (plusieurs dizaines de minutes).

### EXEMPLE 2

Cet exemple illustre, selon un autre mode de réalisation que celui de l'exemple 1, la préparation d'un support fonctionnalisé conforme à l'invention pouvant être représenté par le schéma suivant : 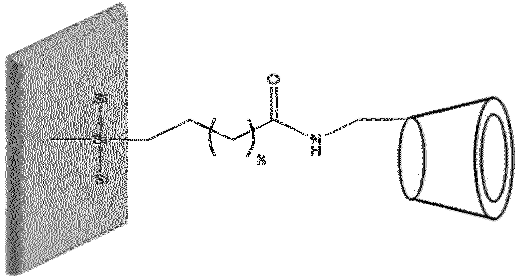 ce support consistant en un support en silicium lié à un groupe β-monoaminocyclodextrine via un groupe espaceur de formule -(CH₂)₁₀-CO-, ce groupe espaceur étant lié au groupe β-monoaminocyclodextrine par l'unique groupe -NH-CH₂- appartenant à ce groupe β-monoaminocyclodextrine.

La préparation de ce support comprend les étapes suivantes :
- la préparation d'un support de silicium fonctionnalisé par un groupe de formule -(CH₂)₁₀-CO-OH (étape a) ;
- le couplage dudit support ainsi fonctionnalisé avec la 6-monoamino-β-cyclodextrine (étape b).

### Etape a-Préparation d'un support en silicium fonctionnalisé par un groupe de formule -(CH₂)₁₀-CO-OH

Le support préparé selon cette étape peut être représenté schématiquement de la façon suivante :

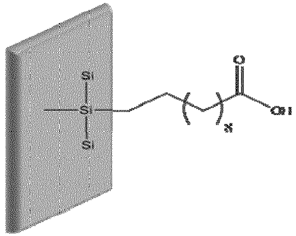

7 mg (3,79*10⁻⁵ mol) d'acide undécénoïque sont pesés et introduits dans un réacteur du type « ACE Pressure Tube ». L'ensemble est ensuite placé dans une boîte à gants sous atmosphère inerte (à savoir, ici, de l'argon comprenant 10 ppm de O₂ et 2 ppm d'eau).

Du mésitylène est distillé en boîte à gants. La teneur en eau (2 ppm) du mesitylène est déterminée par la méthode coulométrique de Karl-Fischer. Quatre morceaux de silicium préalablement traités conformément au protocole exposé à l'exemple 1 sont introduits dans ledit réacteur et 10 mL de mésitylène sont introduits. Le réacteur est alors scellé sous argon. L'ensemble est sorti de la boîte à gants et est mis ensuite à 140°C dans un bain d'huile pendant 24 heures. Les morceaux de silicium ainsi obtenus sont ensuite rincés sous éthanol, sous sonication pendant 10 minutes et séchés sous azote.

### Etape b-Couplage du support obtenu à l'étape a avec la 6-monoamino-β-cyclodextrine

Cette étape b) a été réalisée selon deux protocoles différents (respectivement, un protocole A et un protocole B).

### Protocole A

Les supports préparés selon l'étape a) sont introduits dans un tube de Schlenk sous azote. Parallèlement, 300 mg de N-hydroxysuccinimide (NHS) sont dissous dans 10 mL de diméthylformamide (DMF) anhydre. Après dissolution complète du NHS, la solution est introduite dans le tube sous atmosphère inerte. L'ensemble est alors placé dans la glace pendant 1 heure. 100 µL de diisopropylcarbodiimide sont alors injectés et le tout est remis ensuite à température ambiante.

Après 24 heures, les supports sont retirés du tube de Schlenk et sont rincés avec de la diméthylformamide (trois fois). Ils sont ensuite introduits dans un tube de Schlenk comprenant une solution de 6-monoamino-β-cyclodextrine (100 mg, 8,82*10⁻⁵ mol) dans 5 mL de diméthylformamide et 150 µL de diisopropyléthylamine. Après 24 heures, les supports sont retirés du tube, sont rincés avec de la diméthylformamide (trois fois) puis mis sous sonication dans de l'eau milliQ (3 minutes) puis séchés sous azote.

### Protocole B

Les supports préparés selon l'étape a) sont introduits dans un tube de Schlenk sous azote. 1 mL de chlorure de thionyle SOCl₂ est introduit dans le tube. L'ensemble est maintenu 24 heures à température ambiante. Le surplus est retiré à la seringue puis le tube est mis sous vide poussé pendant 1 heure. Après remise sous azote, une solution de 6-monoamino-β-cyclodextrine (100 mg, 8,82*10⁻⁵ mol) dans 5 mL de formamide et 150 µL de diisopropyléthylamine est introduite dans le tube.

Après 24 heures, les supports sont retirés du tube, sont rincés avec de la diméthylformamide (trois fois) puis mis sous sonication dans de l'eau milliQ (3 minutes) et séchés sous azote.

### EXEMPLE 3

Cet exemple illustre la préparation d'un support fonctionnalisé conforme à l'invention pouvant être représenté par le schéma suivant : 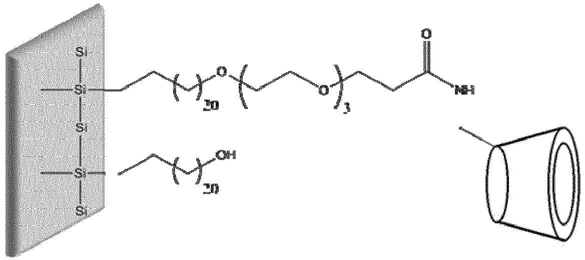 ce support consistant en un support en silicium lié à des groupes β-monoaminocyclodextrines via un groupe espaceur de formule - (CH₂)₂₂-O-(CH₂₋CH₂-O)₃-CH₂-CH₂-CO-, ce groupe espaceur étant lié au groupe β-monoaminocyclodextrine par l'unique groupe -NH-CH₂-appartenant à ce groupe β-monoaminocyclodextrine et ledit support étant lié à des groupes organiques de formule -(CH₂)₂₂-OH non lié à une groupe β-monoaminocyclodextrine.

La préparation de ce support comprend les étapes suivantes :
- la préparation d'un support de silicium fonctionnalisé par des groupes de formule -(CH₂)₂₂-O-(CH₂-CH₂-O)₃-CH₂-CH₂-CO-OtBu (tBu signifiant tertiobutyle) et des groupes de formule -(CH₂)₂₂-O-CO-CH₃ (Etape a) ;
- le couplage dudit support ainsi fonctionnalisé avec la 6-monoamino-β-cyclodextrine (Etape b).

### Etape a-Préparation du support de silicium fonctionnalisé

Cette étape comporte la préparation préalable de deux composés intermédiaires : le 1-O-acétyl-10-O-[1-docos-21-ényl]triéthylèneglycol et l'acétate de docos-21-ényle.

### a1-Préparation du 1-O-acétyl-10-O-[1-docos-21-ényl]triéthylèneglycol

Ce composé est préparé conformément au schéma réactionnel : 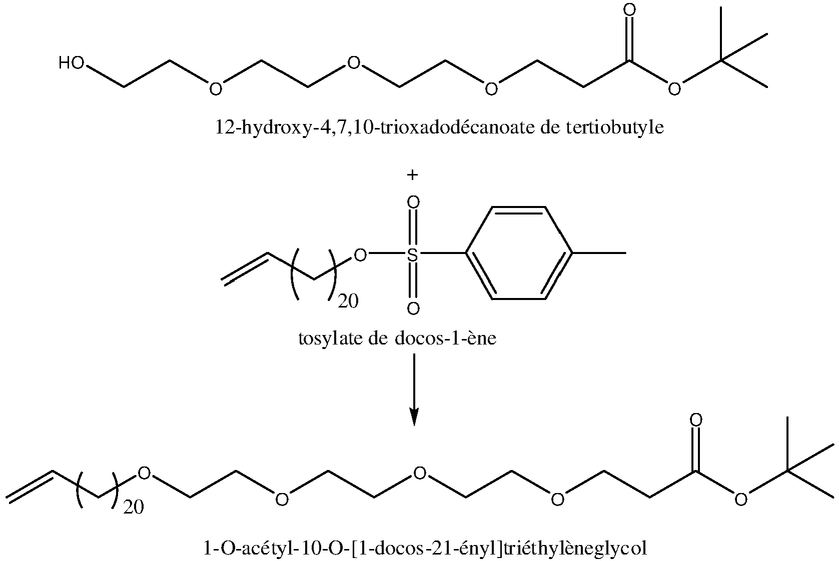 le tosylate de docos-1-ène étant préparé selon le schéma réactionnel suivant :

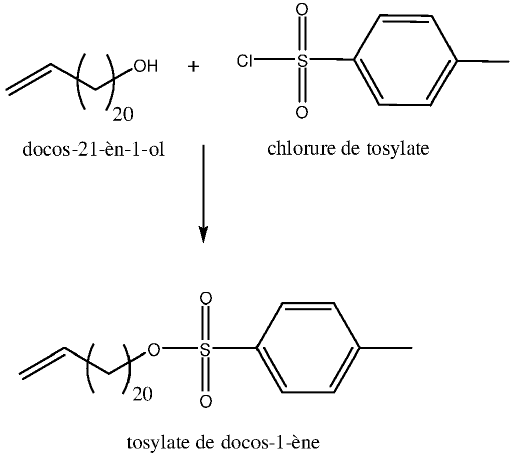

### *Préparation du docos-21-èn-1-ol

### Première étape : Formation du 11 bromomagnésium-1-undécène

A une solution de tétrahydrofurane anhydre contenant 2,7 g (107 mmoles) de magnésium sous forme de tournures est ajoutée, goutte-à-goutte, une solution de 5 g (21,4 mmoles) de 11-bromo-1-undécène dans 21 mL de tétrahydrofurane. Cette réaction est réalisée sous atmosphère inerte. L'exothermie de la réaction est régulée à l'aide d'un bain de glace. Puis 5 mL de dibromoéthane sont ajoutés à ce mélange. La réaction est maintenue active pendant 1 heure et le surnageant est prélevé à l'aide d'une seringue puis placé dans un ballon sous atmosphère inerte.

### Deuxième étape : Formation de l'alcoolate de lithium

A une solution de tétrahydrofurane anhydre sont ajoutés 5,4 g (21,4 mmoles) de 10-bromoundécanol. La solution est placée à -78°C sous atmosphère inerte puis, à l'aide d'une ampoule équipression, est ajouté 0,71 mL (23,45 mmoles) de méthyllithium, le mélange étant ensuite laissé progressivement revenir à température ambiante.

### Troisième étape : Formation du docos-21-èn-1-ol

A la solution contenant le 11-bromomagnésium-1-undécène refroidie à -78°C, on ajoute 0,21 g (1,1 mmole) d'iodure de cuivre. La solution contenant l'alcoolate de lithium préparée lors de la deuxième étape susmentionnée y est ajoutée goutte-à-goutte au moyen d'une canule. Le mélange est agité pendant une heure à cette température puis 15 heures à température ambiante. La réaction est arrêtée par addition de 40 mL d'éthanol absolu. Un précipité noir se forme. Ce dernier est accentué par l'ajout de 3 mL d'acide chlorhydrique à 10%. Après filtration sur un fritté n° 1, la solution limpide obtenue est extraite trois fois par du diéthyléther. La phase éthérée restante est alors lavée avec de l'eau puis avec une solution saturée en NaHCO₃. La phase organique est alors récupérée, séchée sur MgSO₄ puis concentrée par évaporation des solvants sous pression réduite. Le résidu restant est reprécipité dans de l'acétone anhydre. Après filtration sur fritté 4, on obtient 4,86 g de produit, soit un rendement de 70%.
Les résultats de RMN ¹H, de RMN ¹³C et IR sont les suivants.
RMN ¹H (CDCl₃) δ (ppm) : 5,9-5,6 (m, 1H, CH₂=CH-) ; 5,0-4,75 (m, 2H, CH₂=CH-) ; 3,6 (t, 2H, J=8Hz, CH₂OH) ; 2,1-1,9 (m, 2H, =CH-CH₂) ; 1,65-1,50 (m, 2H, CH₂-CH₂-OH) ; 1,3-1,1 (m, 35H, CH₂).
RMN ¹³C (CDCl₃) δ (ppm) : 139 (CH₂CH=) ; 114 (CH₂CH=) ; 63 (CH₂OH) ; 33,8 (=CH-CH₂-) ; 32,0 (CH₂CH₂OH) ; 29, 6-28, 9 (16 CH₂) ; 25,7 (CH₂(CH₂)₂OH).
IR cm⁻¹: 3400 (OH); 2919 (CH₂ asymétrique); 2851 (CH₂ symétrique).

### *Préparation du tosylate de docos-1-ène

A une solution de 4 g (12 mmoles) de docos-21-èn-1-ol préparé préalablement dans 4 mL de triéthylamine anhydre et 30 mL de dichlorométhane anhydre sont ajoutés sous azote et à température ambiante, 4,6 g (24 mmoles) de chlorure de tosylate lui-même étant dilué dans 30 mL de dichlorométhane.

Le mélange jaunâtre est agité pendant 12 heures. Après filtration sur fritté 4 et évaporation des solvants sous pression réduite, le résidu est purifié par chromatographie sur colonne en gel de silice avec, comme éluant, le dichlorométhane à 100%. On obtient 3,4 g de tosylate de docos-1-ène, soit un rendement de 60%.
Les résultats de RMN ¹H, de RMN ¹³C et IR sont les suivants.
RMN ¹H (CDCl₃) δ(ppm) : 7,78-7,29 (dd, 4H, J=83 Hz et 7 Hz, CH=tosyl) ; 5,84-5,47 (m, 1H, CH₂=CH-) ; 5,01-4,85 (m, 2H, CH₂=CH-) ; 3,9 (t, 2H, J=5Hz, CH₂OH) ; 2,43 (s, 3H, CH₃) ; 1,29-1,16 (m, 38H, CH₂-CH₂).
RMN ¹³C (CDCl₃) δ (ppm) : 144, 5 (CCH₃) ; 137,2 (CH₂CH=) ; 133,2 (CS); 130 ((CH=)₂ tosyl); 127,9 ((CH=)₂tosyl); 114,1 (CH₂=) ; 70,6 (CH₂OH) ; 33,7-22,6 (19C, CH₂) ; 21,5 (CH₃).
IR cm⁻¹: 2919 (CH₂ asymétrique); 2851 (CH₂ symétrique) ; 1120 (S=O).

### *Préparation du 12-hydroxy-4,7,10-trioxadodécanoate de tertiobutyle

Dans un bicol de 500 mL contenant du tétrahydrofurane anhydre (250 mL) est ajouté du triéthylèneglycol commercial (80g, 0,47 moles), le tout sous atmosphère d'azote et sous agitation. On ajoute ensuite du sodium (0,4 g, 4,35 mmoles) en attendant sa dissolution complète. On ajoute de l'acrylate de tertiobutyle (21 g, 0,165 mole) à la seringue. Le mélange réactionnel est mis sous agitation pendant 20 heures à température ambiante. Le mélange réactionnel est neutralisé ensuite avec une solution d'acide chlorhydrique (8 mL, 8 mmoles). L'ensemble est agité pendant 30 minutes puis le tétrahydrofurane est évaporé sous pression réduite. La solution est alors versée dans de l'eau salée saturée (100 mL) puis dans de l'acétate d'éthyle (200 mL). La phase organique est alors extraite. Cette opération est renouvelée deux fois. Les différentes phases organiques sont alors rassemblées, séchées puis évaporées sous pression réduire, pour éliminer le solvant. On obtient 42,85 g de composé, soit 93,3 % de rendement.
Les résultats de RMN ¹H et de RMN ¹³C sont les suivants.
RMN ¹H (CDCl₃) δ(ppm) : 1,33 (s, 9H, (CH₃)₃C); 2,39 (t, 2H, CH₂-O-), ³J_{H-H}= 7 Hz); 3, 48-3, 63 (m, 14H, CH₂O).
RMN ¹³C (CDCl₃) δ(ppm) : 27,9 (CH₃)₃C); 36,2 (CH₂COOtBu); 61,5 (CH₂OH); 66,8 (CH₂CH₂COOtBu); 70,2-70,5 ((OCH₂CH₂O)₂); 72,4 (CH₂CH₂OH); 80, 3 (C(CH₃)₃); 170,8 (COO).

### *Préparation du 1-O-acétyl-10-O-[1-docos-21-ényl]triéthylèneglycol

Dans une solution de 10 mL de tétrahydrofurane anhydre sous un flux d'azote contenue dans un premier ballon, on dissout 0,305 g (1,1 mmoles) du 12-hydroxy-4,7,10-trioxadodécanoate de tertiobutyle. Ensuite, on ajoute peu à peu 35 mg d'hydrure de sodium NaH, le mélange résultant étant agité pendant 15 minutes. Dans un autre ballon tricol, on dissout 0,5 g (1 mmole) de tosylate de docos-1-ène dans 7 mL de tétrahydrofurane anhydre. Le contenu de ce ballon est ensuite transvasé goutte-à-goutte dans le premier ballon. Le tout est ensuite agité et chauffé à 80°C pendant 12 heures. On effectue ensuite une extraction au dichlorométhane. La phase organique est lavée trois fois avec une solution aqueuse saturée en hydrocarbonate de sodium. Après séchage avec du sulfate de magnésium MgSO₄ et évaporation des solvants sous pression réduite de la phase organique, on récupère une pâte jaunâtre. Après purification par chromatographie sur colonne en gel de silice avec comme éluant un mélange heptane/acétate d'éthyle (50/50, v/v), on obtient 0,45 g de produit, soit un rendement de 90%.
Les résultats de RMN ¹H et de RMN ¹³C sont les suivants.
RMN ¹H (CDCl₃) δ(ppm) : 1,29-1,16 (m, 38H, CH₂-CH₂) ; 1,33 (s, 9H, (CH₃)₃C); 2,39 (t, 2H, CH₂-O-, ³J_{H-H}= 7 Hz); 2,43 (s, 3H, CH₃); 3, 48-3, 63 (m, 14H, CH₂O); 3,9 (t, 2H, J=5Hz, CH₂O) ; 5,01-4,85 (m, 2H, CH₂=CH-) ; 5,84-5,47 (m, 1H, CH₂=CH-).
RMN ¹³C (CDCl₃) δ(ppm) : 171 (CO) ; 170,8 (COO) ; 139 (CH₂CH-) ; 114 (CH₂CH-) ; 80,3 (C(CH₃)₃) ; 70,2-70,5 ((OCH₂CH₂O)₂); 66,8 (CH₂CH₂COOtBu) ; 64,0 (-CH₂OCO) ; 61,5 (CH₂OH) ; 36,2 (CH₂COOtBu) ; 33,4 (=CH-CH₂-) ; 30,1-29,8 (18C, CH₂); 27,9 (CH₃)₃C); 25,7 (CH₂(CH₂)₂O); 21,5 (CH₃).
IR cm⁻¹= 2919 (CH₂ asymétrique); 2851 (CH₂ symétrique); 1740 (C=O); 1100 (C-O).

### a2-Préparation de l'acétate de docos-21-ényle

A 2g de docos-21-èn-1-ol tel préparé ci-dessus, on ajoute 20 mL de pyridine. Après quelques minutes d'agitation, la solution est refroidie par un bain de glace et 0,7 mL (7,2 mmoles) d'anhydride acétique sont ajoutés. Après retour à température ambiante, la solution est agitée pendant 12 heures. La pyridine est alors co-évaporée à l'aide du toluène anhydre puis le résidu est versé dans 30 mL de dichlorométhane. La solution résultante est lavée trois fois avec une solution aqueuse saturée en hydrocarbonate de sodium. Les phases organiques sont réunies après séchage sur du sulfate de magnésium MgSO₄. Les solvants sont évaporés sous pression réduite. Une poudre blanche est récupérée. Après purification par chromatographie sur colonne en gel de silice avec comme éluant un mélange dichlorométhane/méthanol (98/2, v/v), on obtient 1,34 g de produit, soit un rendement de 62%, sous forme d'une huile translucide, qui durcit et blanchit à température ambiante.
Les résultats de RMN ¹H, de RMN ¹³C et IR sont les suivants.
RMN ¹H (CDCl₃) δ(ppm) : 5,92-5,66 (m, 1H, CH₂=CH-) ; 4,98-4,85 (m, 2H, CH₂=CH-); 4,04-3,98 (t, 2H, J=8Hz, CH₂OH) ; 2,06 (s, 3H, CH₃CO); 1,7-1,5 (m, 2H, CH₂-CH₂-OH) ; 1,29-1,15 (m, 36H, CH₂).
RMN ¹³C (CDCl₃) δ(ppm) : 171,2 (CO) ; 139 (CH₂CH-); 114,2 (CH₂CH-) ; 64 (-CH₂O); 33 (=CH-CH₂-) ; 30,1-29,8 (17 CH₂) ; 25,7 (CH₂(CH₂)₂OH); 20,8 (COCH₃).
IR cm⁻¹ : 2919 (CH₂ asymétrique); 2851 (CH₂ symétrique) ; 1740 (C=O) ; 1220 (C-O).

### a3-Préparation du support fonctionnalisé

7 mg (1,19*10⁻⁵ mol) de 1-O-acétyl-10-O-[1-docos-21-ényl]triéthylèneglycol et 4 mg (1,09*10⁵ mol) d'acétate de docos-21-ényle sont pesés et introduits dans un réacteur du type « ACE Pressure Tube ». L'ensemble est placé dans une boîte à gants sous atmosphère inerte (à savoir, ici, de l'argon comprenant 10 ppm d'oxygène et 2 ppm d'eau).

Le mésitylène est distillé dans la boîte à gants. La teneur en eau (2 ppm) du mésitylène est déterminée par la méthode coulométrique de Karl-Fischer. Quatre morceaux de silicium préalablement traités selon un protocole déjà exposé à l'exemple 1 sont introduits dans le réacteur. Le réacteur est alors scellé sous argon. L'ensemble est extrait de la boîte à gants et est mis à 140°C dans un bain d'huile pendant 24 heures. Les morceaux de silicium ainsi obtenus sont ensuite rincés sous éthanol, placés sous sonication pendant 10 minutes puis séchés sous azote.

### Etape b-Couplage dudit support ainsi fonctionnalisé avec la 6-monoamino-β-cyclodextrine.

Les supports fonctionnalisés obtenus ci-dessus sont soumis à la potasse alcoolique pendant 30 minutes sous sonication. Après rinçage à l'eau milliQ et à l'éthanol, ils sont séchés sous flux d'azote.

L'étape de couplage est réalisée selon le protocole explicité ci-dessous.

Les supports préparés selon l'étape a) et déprotégés grâce au traitement préalable avec la potasse alcoolique sont introduits dans un tube de Schlenk sous azote. Parallèlement, 300 mg de N-hydroxysuccinimide (NHS) sont dissous dans 10 mL de diméthylformamide (DMF) anhydre. Après dissolution complète du NHS, la solution est introduite dans le tube sous atmosphère inerte. L'ensemble est alors placé dans la glace pendant 1 heure. 100 µL de diisopropylcarbodiimide sont alors injectés et le tout est remis ensuite à température ambiante.

Après 24 heures, les supports sont retirés du tube de Schlenk et sont rincés avec de la diméthylformamide (trois fois). Ils sont ensuite introduits dans un tube de Schlenk comprenant une solution de 6-monoamino-β-cyclodextrine (100 mg, 8,82*10⁻⁵ mol) dans 5 mL de diméthylformamide et 150 µL de diisopropyléthylamine. Après 24 heures, les supports sont retirés du tube, sont rincés avec de la diméthylformamide (trois fois) puis mis sous sonication dans de l'eau milliQ (3 minutes) puis séchés sous azote.

### EXEMPLE 4

Cet exemple illustre la préparation d'un support fonctionnalisé conforme à l'invention pouvant être représenté par le schéma suivant : 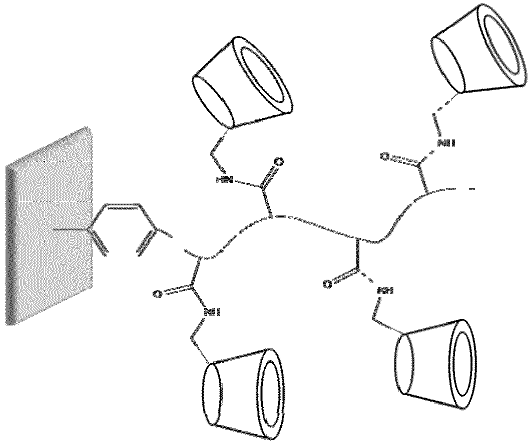 ce support consistant en un support en verre recouvert d'une couche en or allié à du chrome, cette couche étant liée, via un groupe phényle, à un polymère comprenant des unités répétitives de formule suivante : 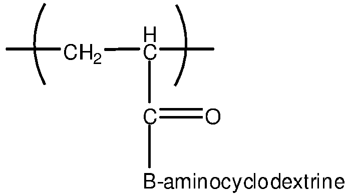 la (β-cyclodextrine étant liée au groupe -CO- au moyen de l'unique groupe -CH₂-NH- appartenant à ce groupe β-monoaminocyclodextrine.

La préparation de ce support comprend les étapes suivantes :
- une étape préalable de préparation du support non fonctionnalisé (étape a) ;
- une étape de préparation d'un support fonctionnalisé, via un groupe phényle, par des chaînes poly(acide acrylique) (étape b) ;
- une étape de couplage du support fonctionnalisé obtenu à l'étape b) avec des composés β-aminocyclodextrine (étape c).

### Etape a-Préparation du support non fonctionnalisé

Des lames de microscope en verre sont, dans un premier temps, lavées sous sonication dans de l'eau (10 minutes), dans l'acétone (10 minutes) puis dans l'éthanol (10 minutes). Les lames sont ensuite séchées sous un flux d'azote et montées sur un substrat en vue d'effectuer le dépôt d'une couche en or allié à du chrome.

L'ensemble (lames+substrat) est introduit sous ultravide (10⁻⁷ Torrs) dans un banc d'évaporation (Balzers BAK600). Sur chaque lame est déposée une couche en or allié au chrome par métallisation à l'or (or à 99,99%, 40 nm) précédée d'une évaporation de 5 nm de chrome.

### Etape b-Préparation du support fonctionnalisé à partir des supports obtenus à l'étape a)

905 mg (5.10⁻³ mol) de 4 amino-aniline sont dissous dans 50 mL d'acide chlorhydrique 2N. En parallèle, on dissout 690 mg (0,01 mol) de NaNO₂ dans 50 mL d'eau milliQ (18 MΩ). La solution de NaNO₂ est ajoutée goutte-à-goutte dans la solution de 4 amino-aniline sous agitation. 6 mL d'acide acrylique sont alors ajoutés à la solution obtenue. 100 mg de limaille de fer sont alors introduits dans la solution. Des lames de verre métallisées (dites supports non greffés) préparées selon l'étape a) sont introduites dans la solution de greffage.

Après 45 minutes, les lames sont sorties de la solution et rincées à l'eau sous sonication (3 minutes). Afin d'éliminer le poly(acide acrylique) non lié, les lames sont placées dans une solution de NaOH à 1N sous sonication pendant 10 minutes. Les lames sont ensuite passées dans une solution d'acide chlorhydrique 1N pendant 10 minutes et séchées sous flux d'azote.

### Etape c-Etape de couplage du support obtenu à l'étape b) avec de la 6-monoamino-β-cyclodextrine

Les supports préparés à l'étape b) sont introduits dans un réacteur sous azote. 300 mg (2,64*10⁻⁴ mol) de 6-monoamino-β-cyclodextrine sont dissous dans 20 mL de diméthylformamide anhydre sous atmosphère inerte. Après dissolution, la solution est injectée dans le réacteur. On ajoute ensuite 1 mL de N-(3-diméthylaminopropyl)-N'-éthylcarbodiimide d'acide dipicolinique à l'aide d'une seringue. Après 48 heures à température ambiante, les supports sont sortis et rincés avec du diméthylformamide (3 fois). Ils sont, par la suite, soumis à un traitement de sonication pendant 10 minutes dans de l'eau milliQ, rincés à l'éthanol et séchés sous flux d'azote.

### EXEMPLE 5

Cet exemple illustre la préparation d'un support similaire à celui de l'exemple 4 selon un autre mode de réalisation.

La préparation de ce support comprend les étapes suivantes :
- une étape préalable de préparation du support non fonctionnalisé (identique à l'étape a) de l'exemple 4) ;
- une étape de préparation d'un composé intermédiaire : le N-mono-6N-β-cyclodextrine-acrylamide (étape a) ;
- une étape de couplage du support obtenu avec les composés préparés à l'étape a) susmentionnée (étape b).

### Etape a-Préparation de la N-mono-6N-β-cyclodextrine-acrylamide

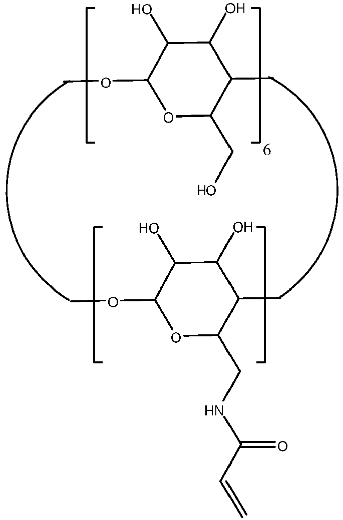

Le protocole précis de préparation de ce composé est le suivant.

19,1 mg (0,264 mmol ; 18,8 µL) d'acide acrylique et 30,38 mg (0,264 mmol) de N-hydroxysuccinimide sont dissous dans 3 mL de diméthylformamide anhydre. L'ensemble est placé dans la glace pendant 30 minutes. 40,98 mg (0,264 mmol ; 46,8 µL) de N-(3-diméthylaminopropyl)-N'-éthylcarbodiimide sont injectés et la solution est ramenée à température ambiante pendant 6 heures. La solution est filtrée sur un fritté du type 4, afin d'éliminer l'urée formée. 300 mg (2,64*10⁻⁴ mol) de N-mono-6N-β-cyclodextrine-acrylamide sont dissous dans 10 mL de diméthylformamide anhydre et ajoutés à la solution d'acide acrylique activée. L'ensemble est agité sous atmosphère inerte pendant 48 heures puis le volume de diméthylformamide est réduit sous pression réduite. Le tout est alors versé dans 100 mL d'acétone, afin d'obtenir un précipité blanc. Le solide obtenu est filtré sur fritté du type 4 et lavé 4 fois avec de l'acétone. Après séchage sous vide, on obtient 261,22 mg du produit (0,22 mol, soit 83% de rendement).
RMN ¹H (DMSO-d⁶) δ(ppm) : 7,89 (m, 1H) ; 6,48 (q, 1H); 6,0 (dd, 1H); 5,52 (dd, 1H); 5,78-5,63 (m, 14H); 4,90-4,85 (m, 7H); 4,50-4,45 (m, 6H); 3,66-3,54 (m, 28H); 3,42-3,24 (m, 16H)

### Etape b-Etape de couplage avec le support

181 mg (1*10⁻³ mol) de 4-aminoaniline sont dissous dans 10 mL d'acide chlorhydrique 2N. En parallèle, 138 mg (2*10⁻³ mol) de NaNO₂ sont dissous dans 10 mL d'eau milliQ (18 MΩ). La solution de NaNO₂ est ajoutée goutte-à-goutte dans la solution de 4-aminoaniline sous agitation. 180 mg (1,51*10⁻⁴ mol) de N-mono-6N-β-cyclodextrine-acrylamide sont ajoutés à la solution ainsi obtenue. 100 mg de limaille de fer sont alors introduits dans la solution. Des lames de verre métallisées de 1 cm² sont introduites dans la solution de greffage.

Après 45 minutes, les lames sont sorties de la solution et rincées à l'eau sous sonication (3 minutes). Les lames sont ensuite passées dans une solution d'acide chlorhydrique HCl 0,1 N pendant 10 minutes et séchées sous flux d'azote.

### EXEMPLE 6

Cet exemple illustre la préparation d'un composé comprenant une séquence peptidique cible d'une enzyme MMP spécifique (l'enzyme MMP-9), ces composés étant aptes aptes à interagir avec les supports fonctionnalisés préparés selon les exemples ci-dessus pour former des complexes « hôte-invité » avec les groupes cyclodextrines portés par lesdits supports fonctionnalisés.

Ce composé répond à la formule suivante :
Lys (DAC)-GPGGVVGP-Lys (MC) A
-GPGGVVGP- correspondant à la séquence peptidique -Gly-Pro-Gly-Gly-Val-Val-Gly-Pro-, Lys(DAC) et Lys(MC) répondant respectivement aux formules suivantes :

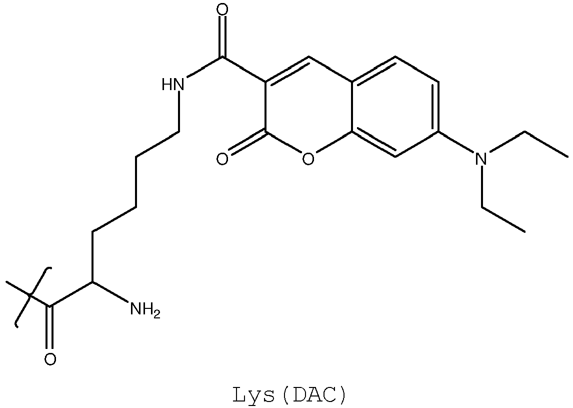

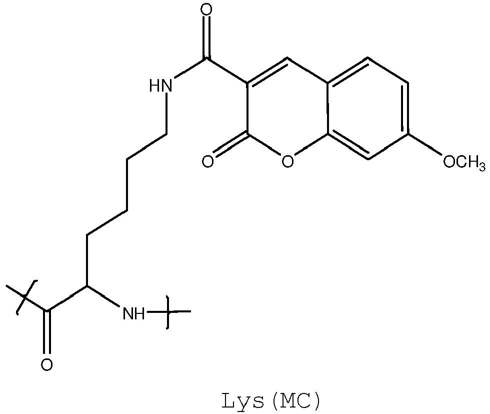

### a) Préparation des composés intermédiaires Fmoc-Lys(DAC)-OH et Fmoc-Lys(MC)-OH

Cette étape illustre la préparation des composés de formule générale suivante : 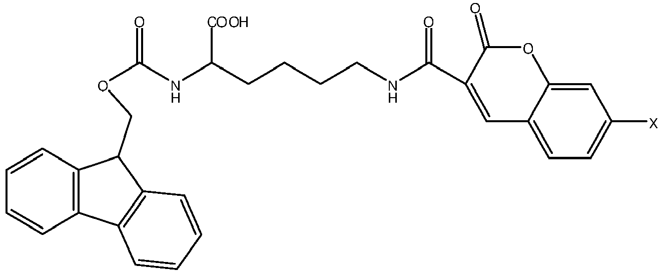 avec X correspondant à un CH₃O- ou Et₂N-,
Fmoc (signifiant fluorophénylméthyloxycarbonyl) correspondant au fragment suivant : 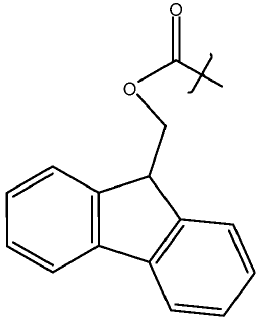 Lys correspondant au fragment suivant : 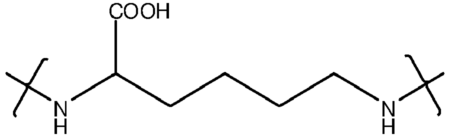 le fragment de formule suivante : 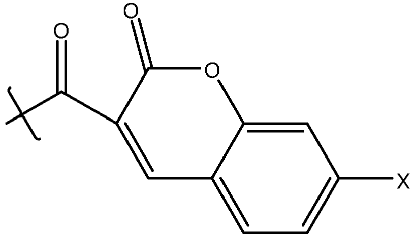 correspondant à l'abréviation MC lorsque X=OCH₃ et à DAC lorsque X= Et₂N-,
le OH dans l'abréviation Fmoc-Lys(MC)-OH ou Fmoc-Lys(DAC)-OH indiquant la présence d'un carboxyle libre dans le fragment -Lys.

Le protocole de préparation est le suivant.

A une solution de Fmoc-Lysine-OH (ce composé étant différent du composé Fmoc-Lys(DAC)-OH par le fait que le groupe -NH- lié au DAC est remplacé par un groupe -NH₂ libre) dans du diméthylformamide (5 mL) sous azote et sous agitation pendant 30 minutes à 0°C, on ajoute un composé (0,2 mole) de formule suivante : 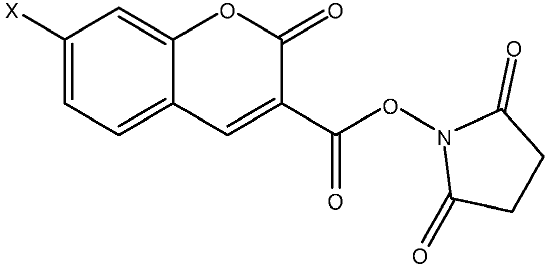 avec X étant tel que défini ci-dessus.

Le mélange est maintenu 30 minutes supplémentaires à 0°C puis laissé 2 heures à température ambiante. Le produit est ensuite précipité avec une solution d'acide chlorhydrique 2N. Après filtration, le solide résultant est lavé avec 4*10 mL d'acide chlorhydrique 2N puis avec 3*10 mL d'eau puis séché à pression réduite, moyennant quoi l'on obtient 581,1 mg de Fmoc-Lys(DAC)-OH (soit un rendement de 95%) et 547,7 mg de Fmoc-Lys(MC)-OH (soit un rendement de 96%).

Pour Fmoc-Lys(MC)-OH, les résultats de RMN sont les suivants.
RMN ¹H (DMSO-*d*₆) δ ppm : 8,73 (1H, s) ; 8,60 (2H, t, J=9,2Hz) ; 7,94-6,81 (10H, m) ; 4,33-4,10 (3H, m) ; 3,85 (3H, m) ; 3,36-3,35 (4H, m) ; 2,72 (1H, s) ; 1,74-1,02 (6H, m).
RMN ¹³C (DMSO-*d*₆) δ ppm : 166,1 ; 164,1 ; 163,0 ; 162,6; 157,8 ; 157,2 ; 149,4 ; 145,7 ; 142,4 ; 133,2 ; 129,3 ; 128,8 ; 127,0 ; 121,8 ; 115,4 ; 113,8 ; 111,6 ; 101,9 ; 67,0 ; 58,0 ; 57,0 ; 48,6 ; 33,9 ; 30,9 ; 28,6.

Pour Fmoc-Lys(DAC)-OH, les résultats de RMN sont les suivants.
RMN ¹H (CDCl₃) δ ppm : 9,01 (1H,s) ; 8,68 (2H, s) ; 7,72-7,22 (8H, m) ; 6,48 (1H, d, J=8,5Hz) ; 6,37 (1H, s) ; 6, 05-5, 96 (2H, m) ; 4,40-4,11 (4H, m) ; 3,52-3,44 (2H, m) ; 3,36 (4H, q, J=6,8Hz) ; 1,95-1,45 (6H, m) ; 1,16 (6H, t, J= 7,5Hz).
RMN ¹³C (CDCl₃) δ ppm : 164,9 ; 163,9 ; 158,7 ; 153,8 ; 145,4 ; 142,4 ; 139,2 ; 132,7 ; 130,4 ; 129,6 ; 128,7 ; 128,3 ; 126,7 ; 120,9 ; 111,2 ; 109,8 ; 97,5 ; 68,0 ; 56, 4 ; 48,5 ; 46,2 ; 40,5 ; 30, 4 ; 23, 6 ; 22,8 ; 13,7.

Le composé de formule suivante: 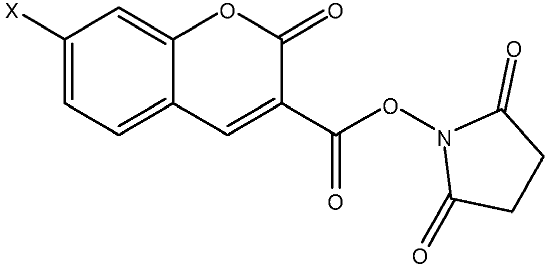 utilisé dans le protocole de préparation des composés Fmoc-Lys(DAC)-OH et Fmoc-Lys(MC)-OH est préparé selon le protocole ci-dessous :
A une solution d'acide 7-méthoxycoumarin-3-carboxylique (pour la préparation du composé pour lequel X=OCH₃) ou bien d'acide 7-diéthylaminocoumarin-3-carboxylique (pour la préparation du composé pour lequel X=-NEt₂) (1 mmole), dans du diméthylformamide (5ml), sous agitation magnétique, du N-Hydroxysuccinimide (115 mg, 1mmole) est ajouté. Le mélange est refroidi à -10°C pendant 1 heure, et du N, N'-dicyclohexylcarbodiimide (226,96 mg, 1,1 mmole) est ajouté. Le milieu est agité une heure à -10°C, puis 3 heures à température ambiante. Le mélange est ensuite filtré pour éliminer le dicyclohexylurée (DCU) et un mélange d'isopropanol/hexane (1/10) est ensuite ajouté afin de précipiter le produit, qui après filtration et séchage sous vide, est sous forme :
   - d'une poudre jaune pour le composé avec X= NEt₂ (251,7 mg ; rendement = 70%), les résultats étant les suivants :
      RMN ¹H (CDCl₃) δ ppm : 8,58 (2H,s) ; 7,38 (1H, d, J=9, 5Hz) ; 6,64 (1H, d, J=9,5Hz) ; 6,45 (1H,s) ; 3,48 (4H, q, J=7,3Hz) ; 2,90 (4H,s) ; 1,26 (6H, t, J=7,3 Hz).
      RMN ¹³C (CDCl₃) δ ppm : 170,8 ; 160,5 ; 160,2 ; 158,4 ; 155,5 ; 152,4 ; 133,3 ; 111,5 ; 109,0 ; 103,8 ; 98,0 ; 46,7 ; 27,0 ; 13,8.
   - d'une poudre blanche pour le composé avec X=-OCH₃ (249,2 mg ; rendement = 78%), les résultats étant les suivants :
      RMN ¹H (DMSO-*d*6) δ ppm : 8,76 (2H,s) ; 7,57 (1H, d, J=8,5Hz) ; 6,95 (1H, d, J=8,5Hz) ; 6,85 (1H,s) ; 3,95 (3H,s) ; 3,34 (2H,s), 2,90 (4H,s).
      RMN ¹³C (DMSO-*d*6) δ ppm : 175,6 ; 161,9 ; 160,2 ; 159,5; 158, 5 ; 153,4 ; 138,3 ; 120,4 ; 117,4 ; 114,3 ; 106,6 ; 62,5 ; 31,8.

### b-Préparation du peptide en tant que tel

Le schéma réactionnel de synthèse de ce peptide est le suivant :

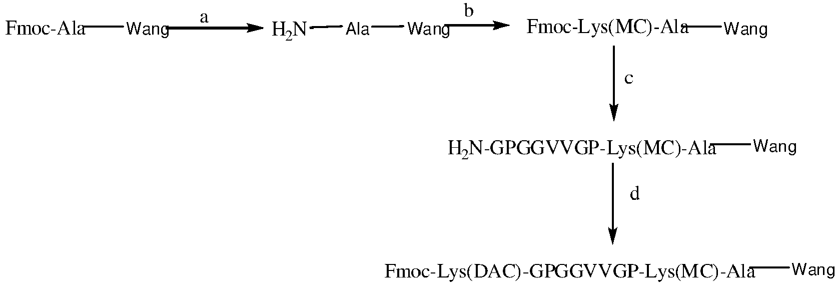

Ainsi, pour la synthèse du peptide susmentionné, on utilise une résine Fmoc-Ala-Wang (0,75 mmole/g).

Selon a) sur le schéma réactionnel, cette résine est déprotégée, et en particulier selon le protocole suivant :
666 mg (0,5 mmole) de résine Fmoc-Ala-Wang ont été mélangés avec 20 ml d'une solution de pepéridine/NMP (50%/50%) pendant trois heures.

Selon b) sur le schéma réactionnel, la résine déprotégée est couplée avec le composé Fmoc-Lys(MC)-OH et en particulier selon le protocole suivant :
La résine précédemment déprotégée a été mélangée avec 286 mg de Fmoc-Lys(MC)-OH (0,5 mmole) en présence de benzotriazol-1-yl-oxytris-pyrrolidinophosphonium (symbolisé par l'abréviation PyBOP) (260 mg, 0,5 mmole), N-hydroxybenzotriazole (symbolisé par l'abréviation HOBt) (67,56 mg, 0,5 mmole), et de diisopropyléthylamine (175,13 µl, 1 mmole) dans le diméthylformamide pendant 5 heures à température ambiante. Le produit est ensuite filtré, lavé avec 5 x 10 ml de DMF, 3 x 10 ml de dichlorométhane (DCM) et 2 x 10 ml de méthanol puis notre résine est séchée sous vide.

A l'issue de ce protocole, il est procédé à la détermination du taux de greffage des résines par mesure UV de la concentration en dibenzofluvène libéré lors du clivage du groupe Fmoc en présence de pipéridine.

Pour ce faire, deux fioles jaugées de 50 ml sont préparées. Dans la première fiole, un volume de 0,5 ml d'une solution de diméthylformamide contenant 20% de pipéridine est ajouté, lequel volume est ajusté avec du diméthylformamide, pour obtenir une solution qui nous servira de référence. Dans la deuxième fiole, 5 mg de résine ont été pesés, puis 0,5 ml de la solution pipéridine (20%) / diméthylformamide est rajouté. Le mélange est alors agité pendant 8 min puis complété avec du diméthylformamide. Ensuite, l'absorbance à 301 nm de la solution contenant la résine est mesurée en soustrayant celle de la solution de référence.

On obtient un taux de substitution de 0,64 mmole/g pour la résine Fmoc-Lys(MC)-Ala-Wang.

Selon c) sur le schéma réactionnel, il est procédé à un couplage des acides aminés sur la résine ainsi modifiée.

Pour ce faire, 390,6 mg de résine modifiée (0,25 mmole) sont ensuite chargés dans le réacteur de synthèse peptidique pour la synthèse automatique. Les acides aminés Nα-Fmoc sont chargés à 1 mmole (Glycine, Proline, valine, alanine) et la synthèse est réalisée en double couplage conditionnel en présence de N-[1H-benzotriazol-1-yl)diméthylamino)méthylène]-N-méthyl-méthaminium hexafluorophosphate N-oxyde (symbolisé par l'abréviation HBTU), de HOBt et de DIEA. Après chaque greffage, un cycle de capping à l'anhydride acétique est réalisé. Une déprotection finale des peptides a été réalisée en fin de synthèse.

Enfin, selon d) sur le schéma réactionnel, il est procédé à un greffage du composé Fmoc-Lys(DAC)-OH préparé préalablement.

Pour ce faire, 305 mg de Fmoc-Lys(DAC)-OH (0,5 mmole) sont mélangés avec l'ensemble résine-peptide (issu de la synthèse automatique) en présence de PyBOP (260,15 mg, 0,5 mmole), HOBt (67,5 mg, 0,5 mmole), et du DIEA (175,1 µl, 1 mmole) dans du diméthylformamide pendant 5 heures à température ambiante. La résine est ensuite filtrée, lavée et séchée.

Au final, le peptide obtenu est déprotégé en N-terminal avec une solution pipéridine 50% dans la NMP. Il est ensuite clivé de la résine sous une forme complètement déprotégée avec une solution de TFA/T.I.S/H₂O (95 : 2,5 : 2,5) pendant 2h30 à température ambiante. La résine est ensuite éliminée par filtration et lavage avec 2 x 1 ml de TFA et 10 ml de DCM. Le filtrat est évaporé à pression réduite et le peptide est précipité avec de l'éther diéthylique froid. On récupère 300 mg de peptide brut par filtration.

Le peptide brut est ensuite purifié par HPLC.

Les mêmes protocoles qu'exposés ci-dessus sont mis en oeuvre pour la réalisation d'un composé comprenant une séquence peptidique cible d'une enzyme du type MMP1, ce composé répondant à la formule suivante :
Lys (DAC)-GPQGLLGA-Lys (MC) A
et d'une séquence peptidique cible d'une enzyme du type MMP-2, ce composé répondant à la formule suivante :
Lys (DAC)-PPGAYHGA-Lys (MC) A

### EXEMPLE 7

Cet exemple illustre la préparation d'un composé comprenant une séquence peptidique cible d'une enzyme MMP spécifique aptes à interagir avec les supports fonctionnalisés préparés selon les exemples ci-dessus pour former des complexes « hôte-invité » avec les groupes cyclodextrines portés par lesdits supports fonctionnalisés, ce composé répondant à la formule suivante :

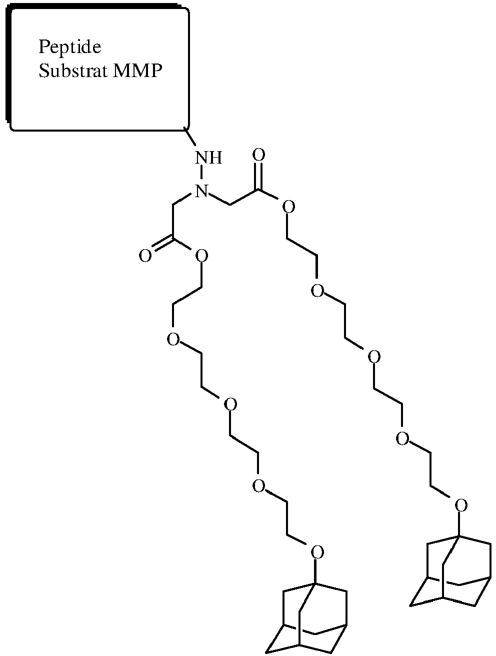

La préparation de ce composé nécessite la préparation de composés intermédiaires, dont les protocoles de préparation sont exposés ci-dessous.

### a-Préparation du composé 1

Ce composé 1 répond à la formule suivante :

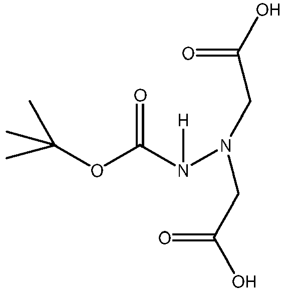

Le protocole précis de préparation de ce composé est le suivant.

Dans un ballon de 100 ml contenant 30 ml de toluène, 1 g de carbazate de terbutyle (7,57 mmoles) ont été ajoutés. Après solubilisation du carbazate, 3,47 g (22,7 mmoles) de bromoacétate de méthyle et 2,08g (15,13 mmoles) de carbonate de potassium ont été additionnés au mélange réactionnel. Le mélange réactionnel a été porté à 90°C pendant 48 heures. Par la suite, le solvant a été évaporé sous pression réduite. Le produit de la réaction n'étant pas pur, il est ensuite purifié par chromatographie sur gel de silice Flash avec comme éluant un mélange dichlorométhae/acétate d'éthyle (90/10, V/V).

Pour effectuer la saponification du produit obtenu, une solution NaOH à 1N a été utilisée en présence du produit dans un mélange de solvant acétonitrile / eau (60% /30%), sous agitation à température ambiante pendant 12h. Ensuite, l'acétonitrile a été évaporé sous pression réduite et un lavage avec du diéthyléther a été réalisé dans la foulée. La phase aqueuse récupérée a été ensuite lyophilisée. Le produit est obtenu sous forme d'un sel de sodium, qui a été acidifié à l'aide d'une solution (30 ml) de HCl à 0.1N. La réaction a été maintenue pendant 2h sous agitation et à température ambiante, suivie d'une extraction avec 50 ml d'acétate d'éthyle réalisée 3 fois. Enfin, un lavage de la phase organique récupérée avec 50 ml d'eau a été effectué. Après évaporation du solvant organique sous pression réduite nous obtenons 1.49 g de produit pur soit un rendement réactionnel de 80%.

Les résultats de RMN ¹H et de RMN ¹³C sont les suivants.
RMN ¹H (MeOD) δ ppm : 1,3 (s, 9H, (CH₃)-Boc) ; 3,35 (s, 4H, (CH₂)₂COOH), 8, 5 (s, NH-N), 10, 23 (s, (CH₂-COOH)₂).
RMN ¹³C (CDCl₃) δ ppm : 27,3, 45,43, 78,51, 153,29, 163,35.

### b-Préparation du composé 2

Ce composé 2 répond à la formule suivante :

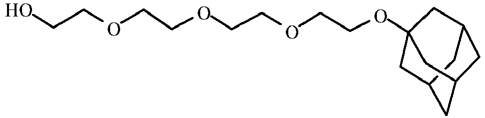

Le protocole précis de préparation de ce composé est le suivant.

Dans un ballon contenant 250 mL de tétraéthylène glycol, on ajoute 15g (69,8 mmoles) de 1-bromoadamantane et 30 mL (216 mmoles) de triéthylamine. Le mélange réactionnel est agité pendant 14 heures à 180°C. Après refroidissement, 250 mL de dichlorométhane ont été ajoutés. La solution a ensuite été lavée avec de l'acide chlorhydrique 2N (4x100ml) puis avec une solution saturée de NaCl (1x100ml). Après séchage de la solution organique sur sulfate de magnésium MgSO₄, le solvant a été évaporé et le produit récupéré sous forme d'une huile brunâtre (21,8g, soit un rendement 95%).

Les résultats de RMN ¹H et de RMN ¹³C sont les suivants.
RMN ¹H (CDCl₃) δ ppm : 3,73 (t, J=4,6 Hz, 2H; AdOCH₂ CH₂), 3, 69-3, 66 (m, 8H; OCH₂CH₂O), 3, 64-3, 58 (m, 6H; AdOCH₂CH₂+CH₂CH₂OH), 2,89 (s, 1H; CH₂OH), 2,15 (m, 3H; CH₂CHCH₂[Ad]), 1,75-1,76 (m, 6H; CHCH₂C[Ad]), 1,67-1,57 (m, 6H; CHCH₂CH[Ad]).
RMN ¹³C (CDCl₃) δ ppm : 72,0, 71,8, 70,8, 70,1, 70,0, 69,8, 61,2, 58,7, 40,9, 35,9, 30,0.

### c-Couplage du composé 1 avec le composé 2

Le composé de couplage du composé 1 avec le composé 2 est un composé de formule suivants :

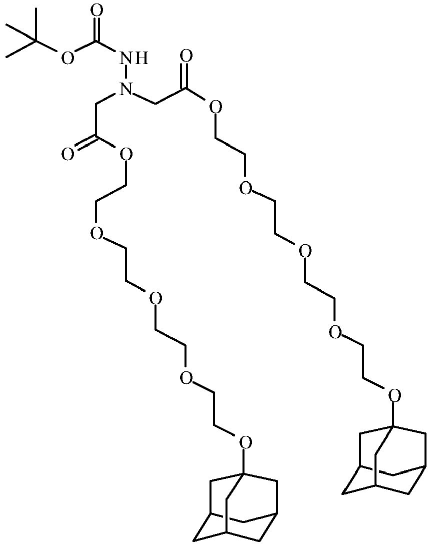

Dans un ballon de 50 mL, on dissout 1g (4,05 mmoles) du composé 1 dans 15 mL de diméthylformamide. Ensuite 2,18 g de N-hydroxybenzotriazole (HOBt) (16,2 mmoles) et 2,55g (20,25 mmoles) de diisopropylocarbodiimide ont été ajoutés, suivi de 2,66g (8, 1 mmoles) du composé 2 et 0,81g (8,1 mmoles) de triéthylamine. Le mélange réactionnel est agité pendant 12 heures à température ambiante sous atmosphère d'azote. Le solvant a été ensuite évaporé sous pression réduite. 100 ml de dichlorométhane ont été ensuite ajoutés au produit obtenu et un lavage avec 30 mL d'eau est effectué 2 fois. Enfin après évaporation du dichlorométhane, une purification par chromatographie Flash sur gel de silice est réalisée en utilisant un mélange d'éluant dichlorométhane/méthanol (90/10 v/v). Le produit est récupéré sous forme d'une huile jaunâtre (2,85 g, soit 81% de rendement).

Les résultats de RMN ¹H et de RMN ¹³C sont les suivants.
RMN ¹H (CDCl₃) δ ppm 6,83 (s, 1H, NH-N) ; 4,28 (s, 4H,(CH₂-COO-)₂) ; 3,86 (s, 4H, (CH₂)₂-N-NH) ; 3,72-3,59 (m, 28H, CH₂-O-CH₂); 2,15 (s, 6H, (CH₂CHCH₂[Ad])₂) ; 1,75 (s, 12H, (CHCH2C[Ad])₂) ; 1,67-1,62 (m, 12H, (CHCH2CH[Ad])₂), 1,45 (s, 9H, (CH₃)₃C).
RMN ¹³C (CDCl₃) δ ppm : 171,22 ; 149,37 ; 79,33 ; 73,92 ; 73,6 ; 72,6 ; 71,98 ; 71,92 ; 71.73 ; 70,21 ; 65,29 ; 63,04 ; 60,63 ; 58,41 ; 42,85 ; 37,79 ; 31,82 ; 29,68.

### d-Déprotection de la fontion amine du composé 3

Le composé préparé selon cette étape est un composé 4 de formule suivants :

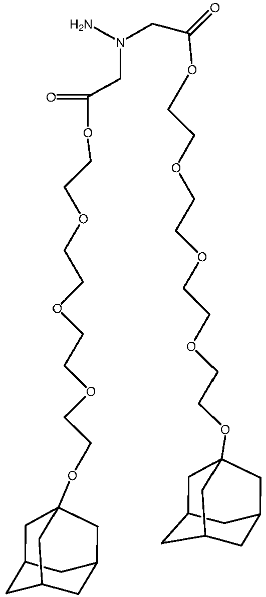

Le protocole précis de préparation de ce composé est le suivant.

1g (1,15 mmoles) du composé 3 est solubilisé dans 10 ml d'un mélange dichlorométhane/acide trifluoroacétique (7/3 v/v) . La réaction de déprotection est maintenue pendant 2 heures à température ambiante. Par la suite, on neutralise le pH du mélange réactionnel en ajoutant du NaHCO₃ en poudre. Une fois la neutralité atteinte, on extrait le produit avec du dichlorométhane. Enfin, la phase organique est séchée sur du sulfate de magnésium MgSO₄ et le solvant est alors évaporé sous pression réduite. 884 mg de produit sont alors récupérés sous forme de poudre, ce qui donne un rendement réactionnel de 100%.
RMN ¹H (CDCl₃) δ ppm : 6,83 (s, 1H, NH-N) ; 4,28 (s, 4H, (CH₂-COO-)₂) ; 3,86 (s, 4H, (CH₂)₂-N-NH) ; 3,72-3,59 (m, 28H, CH₂-O-CH₂) ; 2,24 (bs, 2H, NH₂) ; 2,15 (s, 6H, (CH₂CHCH₂[Ad])₂) ; 1,75 (s, 12H, (CHCH2C[Ad])₂) ; 1,67-1,62 (m, 12H, (CHCH2CH[Ad])₂).
RMN ¹³C (CDCl₃) δ ppm : 171,22 ; 73,92 ; 73,6 ; 72,6 ; 71,98 ; 71,92 ; 71.73 ; 70,21 ; 65,29 ; 63,04 ; 60,63 ; 58,41 ; 42,85 ; 37,79 ; 31,82.

### e-Préparation des peptides destinés à être couplés avec le composé 4

Les peptides préparés selon cette étape sont les peptides suivants:
**S1** :Fmoc-Lys(DAC)-Gly-Pro-Gln-Gly-Leu-Leu-Gly-Ala-Lys(MC)-Ala-CO₂H
   Masse molaire: 1692,54 g/mole
**S2**: Fmoc-Lys(DAC)-Pro-Pro-Gly-Ala-Tyr-His-Gly-Ala-Lys(MC)-Ala-CO₂H
   Masse molaire: 1749,46 g/mole
**S3**: Fmoc-Lys(DAC)-Gly-Pro-Gly-Gly-Val-Val-Gly-Pro-Lys(MC)-Ala-CO₂H
   Masse molaire: 1619,55 g/mole
les fragments Fmoc-Lys(DAC) et Lys(MC) correspondant aux formules telles que fournies à l'exemple 6, le -CO₂H indiquant que le reste d'alanine comporte un groupe -CO₂H libre.

Ces peptides sont préparés selon des protocoles similaires à ceux de l'exemple 6.

### f-Couplage des peptides préparés à l'étape e) avec le composé 4

Le peptide (S1, S2 ou S3) est dissous dans 1 mL de diméthylformamide à température ambiante. 1 équivalent de HOBt est ajouté et la solution est mise à 0°C sous agitation. Après 30 minutes, 1,1 équivalents de PyBOP sont ajoutés et la solution est ramenée à température ambiante. Après 30 minutes, une solution de composé 4 dans du diméthylformamide (1 équivalent par rapport au peptide) en présence de DIEA (1 équivalent par rapport au peptide) est ajouté au goutte à goutte. Après 12 heures d'agitation à température ambiante, le filtrat est évaporé à pression réduite et le peptide est précipité avec de l'éther diéthylique froid.

Les composés couplés ainsi obtenus (respectivement Sb1, Sb2 et Sb3) sont purifiés en utilisant le même protocole décrit précédemment, Sb1, Sb2 et Sb3 comprenant respectivement une séquence peptidique cible des enzymes MMP-1, MMP-2 et MMP-9.

### EXEMPLE 8

Des essais enzymatiques ont été réalisés avec des composés préparés selon l'exemple 7.

Des solutions stocks des composés couplés préparés à l'exemple 7 (respectivement Sb1, Sb2 ou Sb3) sont préparées dans du diméthylsulfoxide (DMSO) (3mg de peptide dans 200 µL de DMSO).

La concentration des différentes solutions de peptides substrats des enzymes MMP-1, -2 et -9 sont calculées en utilisant le coefficient d'extinction molaire des 2 dérivés des coumarines présentes dans ces peptides (DAC et MC) (λ₃₆₀ₙₘ (MC) = 18500 M⁻¹.cm⁻¹ and λ₄₃₆ₙₘ (DAC)= 26300 M⁻¹.cm⁻¹).

Les solutions d'association sont réalisées en prenant un aliquot de 5 µl des solutions stock dans 2 ml de tampon TRIS à 0,1 N.

Les différents supports fonctionnalisés des exemples 3 et 4 sont introduits dans les différentes solutions stocks Sb1, Sb2 ou Sb3 à l'abri de la lumière et à température ambiante. Après 5 minutes, ils sont rincés trois fois avec une solution de TRIS 0,1 N puis 3 fois à l'eau milliQ.

Les différents complexes sont ensuite étudiés grâce à un microscope d'épifluorescence (Olympus BX61). Avant association aucune fluorescence n'est observée (cube U-MNUA2 : Exc 360-370 nm - Em 420-460 nm.) lors d'une excitation à 360-370 nm (longueur d'onde d'excitation de la MC).

Après association, une émission de fluorescence dans le bleu a été observée (présence de la coumarine MC) sur la surface des supports.

Afin de vérifier l'association dans le temps entre les composés couplés Sb1, Sb2 ou Sb3 et les supports, les supports présentant les composés couplés Sb1, Sb2 ou Sb3 ont été placés dans 2mL d'une solution de 0,1 M de TRIS, 0,1 M de NaCl et 10 mM de CaCl₂ (pH 7,4) à température ambiante. Des aliquots de 20 µL ont été prélevés à intervalle régulier et analysés par fluorescence (spectre d'émission) à 2 longueurs d'excitation 360 nm (MC) et 405 nm (DAC). Aucune variation notable de la fluorescence n'a pu être détectée sur 3 heures montrant ainsi que le complexe peptide/support est stable.

Pour les tests enzymatiques, la solution tampon utilisée est composée de 0,1 M de TRIS, 0,1 M de NaCl, 10 mM de CaCl₂ et 0.05% d'Igepal(CA-630).Le pH de la solution est 7,4.

Les enzymes MMP-1, -2 et -9 sont reçues sous forme inactives (forme zymogène). Préalablement, ces enzymes sont activées en utilisant le protocole suivant : 20 µL d'un aliquot de 220 nM de MMP-1, ou de MMP-2 ou de MMP-9 sont activés avec 10 µM d'une solution d'acétate de 4-aminophenylmercurique (APMA) (TRIS 0,1 M pH=9) à 37°C pendant 3 h.

Chaque support présentant les composés couplés Sb1, Sb2 ou Sb3 est placé dans une cellule en quartz pour mesurer la fluorescence résultant du relarguage en solution après hydrolyse des peptides de la partie contenant la coumarine DAC. En raison de la construction des composés couplés Sb1, Sb2 et Sb3, la partie contenant la coumarine MC reste associé au support présentant les cyclodextrines à sa surface.

Chaque essai enzymatique est réalisé dans un volume de 2 mL avec une concentration de 2 nM de MMP activée. Après l'ajout de l'enzyme activée, le taux initial d'hydrolyse du substrat est suivi par l'augmentation de l'émission de fluorescence à 460 nm (excitation à 405 nm) (émission de la coumarine DAC).

Les vitesses initiales obtenues ont permis de déterminer les constantes de second-ordre Kcat/Km de Sb1, Sb2 ou Sb3 pour leurs enzymes respectives (MMP-1, MMP-2 et MMP-9). L'hydrolyse des peptides a pu être suivie au cours du temps.

Les résultats résultant de la moyenne des résultats obtenus avec les supports des exemples 3 et 4 sont les suivants :

| Composé/Enzyme | Kcat/Km (M⁻¹.s⁻¹) sur support |
|---|---|
| Sb1/MMP-1 | 0,7.10⁵ |
| Sb2/MMP-2 | 2,31.10⁵ |
| Sb3/MMP-9 | 1,27.10⁴ |

Après hydrolyse complète, les supports sont rincés à l'eau milliQ puis séchés. Les différents supports sont ensuite étudiés grâce à un microscope d'épifluorescence (Olympus BX61- cube U-MNUA2 : Exc 360-370 nm - Em 420-460 nm. Une émission de fluorescence dans le bleue (420-460 nm) lors d'une excitation à 360-370 nm (longueur d'onde d'excitation de la MC) apparaît sur les images attestant de la présence du morceau peptidique contenant la coumarine MC à la surface du support.

Les supports après hydrolyse sont ensuite soumis à une solution de β-cyclodextrine (10 µM) et de TRIS 0,1 N à température ambiante pendant 5 minutes. Ils sont par la suite rincés avec la même solution puis une solution TRIS 0,1 N et de l'eau milliQ. Les différents supports sont ensuite étudiés grâce à un microscope d'épifluorescence (Olympus BX61- cube U-MNUA2 : Exc 360-370 nm - Em 420-460 nm). Aucune émission de fluorescence dans le bleu (420-460 nm) lors d'une excitation à 360-370 nm (longueur d'onde d'excitation de la MC) n'est apparue sur les images attestant ainsi de la régénération de la surface du support.

### EXEMPLE 9

Des essais ont été réalisés avec des supports porteurs de groupe β-cyclodextrine (les supports préparés selon les exemples 1 à 5) et des substrats d'enzymes MMP préparés selon l'exemple 6.

Tous les essais ont été réalisés dans un tampon comprenant 0,1 M de TRIS, 0,1 M NaCl, 10 mM CaCl₂, 0,05 % d'Igépal (CA-630), pH=7,4.

Les spectres de fluorescence ont été réalisés à l'aide d'une cuve de 1cm de trajet optique, dans un volume total de 2 mL, avec des largeurs de fente de 10 nm en émission et 10 nm en excitation.

Les enzymes commerciales MMP-1, MMP-2 et MMP-9 ont été mises sous forme d'aliquotes pour être conservées à -80 °C (20 µl d'enzyme à une concentration de 220 nM dans un tampon contenant 50% de tampon TRIS pH=7,4 et 50 % de glycérol distillé deux fois). Chaque aliquote est ensuite activé avec 2 µL d'une solution d'acétate d'aminophénylmercurique (APMA) à 10 µM pendant 3 h à 37 °C. La solution d'APMA doit être réalisée dans un tampon TRIS 0,1 M à pH=9. Le volume de la solution d'APMA doit être à un ratio (1/10) par rapport au volume total de la solution d'activation de l'enzyme.

2 mg de composés (tels que définis à l'exemple 6) ont été solubilisés dans 2 ml de tampon TRIS à pH 7,4. Les supports préparés selon les exemples 1 à 5 ont été plongés dans des solutions de peptides susmentionnés. Ensuite les supports ont été rincés avec des jets d'eau Milli-Q, afin d'éliminer les peptides adsorbés en surface et ne laisser que ceux fixés par liaisons non covalentes aux cavités formées par les groupes β-cyclodextrine. Ensuite, ces supports ont été placés au fond d'une cuve en quartz en présence de 2 mL de tampon TRIS à pH 7,4 et de l'enzyme MMP à une concentration de 2 nm. Les variations de fluorescence des coumarines DAC à 475 nm (excitation à 430 nm) et MC à 401 nm (excitation à 350 nm) ont été suivies au cours du temps.

En observant l'évolution de la fluorescence des groupes DAC et MC portés par les composés fixés sur les supports, l'on constate que la fluorescence du groupe MC augmente en fonction du temps tandis que la fluorescence du groupe DAC reste stable en fonction du temps.

Ces résultats prouvent que, lors du clivage enzymatique, la partie du peptide libérée en solution contient la coumarine MC, et qu'en contrepartie, la partie contenant la coumarine DAC reste fixée à la plaque. Ceci confirme donc que la DAC est la coumarine complexée à la β-cyclodextrine qui permet d'immobiliser le peptide sur la plaque par liaison non covalente.

Après la digestion des peptides fixés sur les plaques, une solution aqueuse d'adamantane à 100 mM a été préparée. Les plaques ont été mises au fond des cuves en quartz en présence de 2 ml de la solution d'adamantane. Les variations de fluorescence des coumarines DAC et MC ont été alors mesurées à l'aide du spectrofluorimètre.

L'adamantane possède une affinité plus importante pour la cavité hydrophobe de la β-cyclodextrine que celle de la DAC ou de la MC. Ainsi les adamantanes présentes en solution vont chasser les coumarines des cavités des β-cyclodextrine. Ainsi les coumarines seront libérées en solution et leur fluorescence pourra être mesurée.

Les mesures de fluorescence ont pu montrer une fluorescence importante et significative de la DAC en solution après relargage de la cavité des β-cyclodextrine des fragments peptidiques après digestion enzymatique. Concernant la fluorescence de la MC, il a été constaté, qu'en excitant à 350 nm le spectre de la fluorescence obtenu correspond à celui du peptide entier. En effet, le phénomène de FRET s'observe (deux pics de fluorescence à 401 nm et à 470 nm). Ceci est dû à la présence de quelques peptides fixés sur la plaque et qui ont échappé à la digestion enzymatique. Mais le nombre de ces peptides non digérés est très limité. La valeur de la fluorescence observée en excitant la MC est très basse en particulier en comparaison avec celle de la DAC.

La fluorescence de la DAC (excitation : 410 nm, émission : 475 nm) passe d'une valeur presque nulle à une valeur de 100 UA après ajout de la plaque avec les peptides digérés. Ceci confirme une fois de plus que les fragments de peptides accrochés aux β-cyclodextrine contiennent bien la DAC et non la MC.

Tous ces résultats prouvent que les peptides s'insèrent bien dans la couche de β-cyclodextrine greffée sur les supports susmentionnés.

Les hydrolyses enzymatiques ont bien montré l'efficacité du système utilisé pour la détection des activités des MMP en solution.

Pour confirmer la régénérabilité des supports, les supports susmentionnés ayant été utilisés ont été mis dans une solution aqueuse de β-cyclodextrine à une concentration de 20 mM. Les supports ont été ensuite rincés avec de l'eau Milli-Q et des images de fluorescence de la surface des supports ont été réalisées avec un microscope d'épifluorescence.

Le passage des supports avec les peptides digérés dans une solution de β-cyclodextrine entraîne la disparition totale de la fluorescence à la surface de ces supports. Ceci prouve bien que les cavités des β-cyclodextrine ont été libérées et que ces supports sont bien réutilisables pour d'autres tests d'activités métalloprotéasiques.

### EXEMPLE 10

Afin de valider l'efficacité des biocapteurs de l'invention, il a été réalisé des essais enzymatiques où les biocapteurs de l'exemple 4 ont été incubés avec des cellules cancéreuses invasives issues de cultures cellulaires, plus précisément, une lignée HeLa issue du cancer du col de l'utérus. Ces cellules sont agressives, elles possèdent un grand pouvoir métastasique et sont très invasives. Elles sont connues pour surexprimer plusieurs types de MMP dont la MMP-1, -2 et -9.

Ces cellules ont été préparées par culture cellulaire selon le protocole suivant.

Les lignées cellulaires utilisées sont toutes cultivées en monocouche sur boîte de Pétri ou en flacon (Nunc). Les lignées Hela sont cultivées, en particulier, dans un milieu nutritif liquide de Eagle modifié par Dulbecco (DMEM) contenant 4.5 g/l de glucose, de la glutamine, auquel il est ajouté avant utilisation 10 % de sérum foetal de veau (SVF) préalablement décomplémenté par 30 minutes de chauffage à 56°C. Les cellules sont cultivées à 37°C, en atmosphère humide contenant 5 % de CO₂ en renouvelant périodiquement tous les 2 à 3 jours les milieux de culture. Lorsqu'elles arrivent à un état de subconfluence, les cellules sont systématiquement repiquées. Pour cela, les cellules sont détachées de leur support par une incubation avec de la trypsine pendant 5 minutes à 37°C. L'action de la trypsine est inhibée par le rajout de milieu complet. Le nombre de cellules récupérées est estimé entre 5 et 6 millions. Un volume de la suspension cellulaire (dépendant de la lignée) est réensemencée sur un nouveau support en présence d'un milieu neuf.

Après culture, les cellules ont été lavées avec un tampon PBS, puis décollées de la boite de culture à l'aide d'une solution de trypsine. Après les avoir centrifugées, un culot de cellules a pu être obtenu et dilué dans un tampon TRIS à pH 7,4.

En parallèle, les biocapteurs synthétisés ont été également incubés dans des cuves de fluorescence avec 20 µL d'extraits protéiques à une concentration en protéines de 4 µg/µL obtenus à partir de cellules cancéreuses de lignée HeLa. Pour ce faire, les culots cellulaires sont remis en suspension dans le tampon de lyse FT (KCl 600mM ; Tris-HCl 20 mM pH 7,8 ; Glycérol 20%), dans lequel est ajouté un inhibiteur de phosphatase (Sigma). La lyse cellulaire est provoquée par un choc thermique comportant trois étapes successives de congélation dans l'azote liquide suivie d'une décongélation à température ambiante. La solution de lyse est centrifugée pendant 15 minutes à 13000 rpm à 4°C et le surnageant est récupéré.

L'évolution de la fluorescence des coumarines MC des composés préparés selon l'exemple 6 a été suivie pendant 1 heure à 37 °C.

Ces essais ont montré une évolution importante de la fluorescence de la MC au cours du temps lors des deux expériences (cellules et extraits protéiques). Ceci prouve donc que les peptides ont été reconnus et clivés par les MMP présents dans les cellules HeLa et leurs extraits protéiques. La constatation qui ressort de ces expériences est que les biocapteurs conçus sont capables de détecter l'activité et donc la présence des MMP dans un milieu cellulaire, tout en sachant que ce milieu ne contient qu'un nombre limité de cellules (1. 10⁶ dans 2 mL). D'un autre coté, il a été démontré l'efficacité des biocapteurs de l'invention à détecter les activités et donc la présence des MMP dans un mélange complexe de protéinases issues de cellules cancéreuses.

### EXEMPLE 11

Après avoir détecté les activités des MMP dans des solutions contenant des cellules à caractère invasif et aussi dans des solutions contenant les extraits protéiques de ces cellules, l'objectif a été de confirmer l'efficacité des biocapteurs de l'invention en les incubant avec des cellules cancéreuses à caractère non-invasif et aussi avec leurs extraits protéiques. Pour cela, il a été choisi une ligné cellulaire HUh7 issue d'un carcinome hépatocellulaire (cancer du foie). Cette lignée cellulaire n'est pas invasive, les cellules HUh7 exprimant peu les MMP, surtout la MMP-2 et la MMP-9, qui sont quasiment absentes dans les cultures. Cette lignée représente donc un test témoin qui peut donner une idée de l'efficacité des biocapteurs de l'invention à distinguer des cellules cancéreuses invasives d'autres cellules cancéreuses moins agressives et moins invasives par le biais de l'expression des MMP.

Ces cellules ont été préparées préalablement par culture cellulaire selon un protocole similaire à celui utilisé pour l'exemple 10.

Les biocapteurs synthétisés de l'exemple 4 ont été incubés pendant 1 heure avec des cellules extraites après culture de la lignée HUh7. Chaque biocapteur a été mis en contact avec un nombre de cellules estimé entre 8. 10⁵ et 1. 10⁶. Le volume total dans lequel s'est déroulé chaque essai a été de 2 mL. Les biocapteurs et les cellules ont été donc mises en contact dans du tampon TRIS à pH 7,4 et l'ensemble a été placé à 37°C. Les variations de la fluorescence de la MC (exc : 350 nm, em : 401 nm) présent sur les composés décrits dans l'exemple 6 au cours du temps ont été enregistrées. Le protocole expérimental est le même que celui employé pour les tests enzymatiques et cellulaires précédents.

Afin de valider la spécificité et l'efficacité des biocapteurs de l'invention, il a été procédé également à des essais enzymatiques en soumettant les biocapteurs à des extraits protéiques réalisés à partir des cellules HUh7. En effet, les extraits protéiques permettent de récupérer les enzymes intracelluaires qui n'étaient pas en contact avec les biocapteurs lors des essais réalisés avec les cellules HUh7.

Ces extraits protéiques ont été préparés préalablement selon le protocole suivant.

Pour ce faire, des cellules sont cultivées dans des boîtes de pétri de 10 cm de diamètre de façon à obtenir suffisamment d'extraits protéiques pour l'analyse des protéines d'intérêt. Les cellules sont lavées avec du PBS, puis trypsinées avant d'être centrifugées à 1500 rpm pendant 5 minutes. Après centrifugation, les culots cellulaires sont remis en suspension dans le tampon de lyse FT (KCl 600 mM ; Tris-HCl 20 mM pH 7,8 ; Glycérol 20%) dans lequel est ajouté un inhibiteur de phosphatase (Sigma). La lyse cellulaire est provoquée par un choc thermique comportant trois étapes successives de congélation dans l'azote liquide suivie d'une décongélation à température ambiante. La solution de lyse est centrifugée pendant 15 minutes à 13000 rpm à 4°C et le surnageant est récupéré.

Ces essais ont été réalisés suivant le même protocole que celui utilisé pour les tests précédents. Chaque biocapteur a été mis en contact avec 20 µl d'une solution d'extrait protéique à une concentration de 4µg/µl. Le volume de tampon TRIS dans lequel se sont déroulés les tests est de 2 ml. Les cuves de fluorescence abritant les essais ont été placées à 37 °C et les variations de la coumarine MC (exc : 350 nm, em : 401 nm) ont ensuite été enregistrées sur une durée d'1 heure.

Les variations de fluorescence de la MC enregistrées pendant 1 heure ont montré que les composés de l'exemple 6 n'ont pas été clivés.

Les cellules HUh7 n'exprimant pas de MMP, elles n'ont donc pas libéré les enzymes capables de reconnaître et de cliver les composés de l'exemple 6 fixés sur support. Ces résultats obtenus sont très intéressants étant donné le fait que ces cellules expriment très peu les MMP, moyennant quoi les peptides substrats de ces enzymes n'ont pas été clivés.

Ces résultats correspondent parfaitement aux attentes par rapport à l'objectif de la conception des biocapteurs et de leurs applications. En effet, les biocapteurs sont capables de déceler la présence des MMP dans une suspension de cellules cancéreuses. La spécificité des biocapteurs à leur incorporation de peptides spécifique des MMP est cruciale à ce niveau d'évaluation puisque aucune activité d'autres protéases n'a parasité nos essais avec les cellules.

Les essais avec les extraits des cellules HUh7 n'ont pas montré une évolution de la fluorescence de la MC au cours du temps, ce qui prouve que les peptides n'ont pas été clivés.

Encore une fois ces résultats permettent de valider la sélectivité et l'efficacité des biocapteurs conçus selon l'invention. Malgré la présence de plusieurs types de protéases dans l'extrait protéique des cellules HUh7, les composés de l'exemple 6 fixés en surface des biocapteurs n'ont pas été clivés. Ce résultat est bien en concordance avec les caractéristiques des cellules de la lignée HUh7, qui sont des cellules cancéreuses non-invasives n'exprimant que très peu de MMP.

## Revendications

1. Biocapteur pour la détection de la présence et/ou de l'activité enzymatique d'au moins une protéase, qui correspond à un biocapteur comprenant un support fonctionnalisé par des composés organiques comprenant au moins un groupe formant cage, lequel groupe formant cage est complexé avec au moins un groupe d'un composé comprenant une séquence peptidique cible de la ou les protéases dont on veut déterminer la présence et/ou l'activité enzymatique, lequel biocapteur est régénérable.

2. Biocapteur selon la revendication 1, dans lequel le support est un support solide inorganique.

3. Biocapteur selon la revendication 2, dans lequel le support solide inorganique comprend un matériau choisi dans le groupe constitué par les verres, les quartz, les céramiques, les métaux et les métalloïdes.

4. Biocapteur selon l'une quelconque des revendications précédentes, dans lequel les composés organiques consistent en un bras espaceur lié par une des ses extrémités au support et par au moins une autre de ses extrémités à au moins un groupe formant cage.

5. Biocapteur selon la revendication 4 dans lequel le groupe formant cage est un groupe β-cyclodextrine.

6. Biocapteur selon la revendication 5, dans lequel le groupe β-cyclodextrine est un groupe cyclique comprenant, dans son cycle, au moins un motif répétitif de formule (I) suivante : 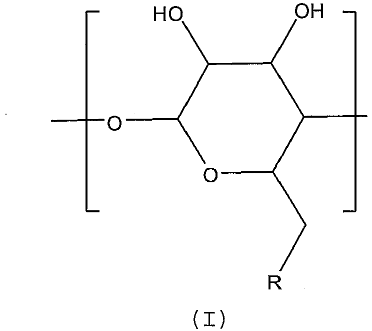 dans laquelle R représente un groupe hydroxyle ou un groupe amino,
et au moins un motif de formule (II) suivants : 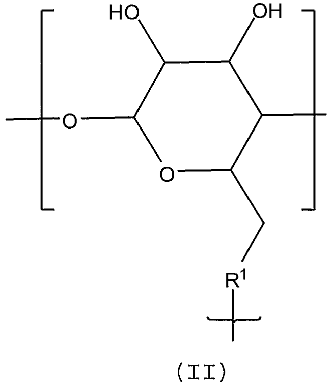 R¹ correspondant à un atome d'oxygène ou à un groupe amino,
l'accolade indiquant l'endroit par lequel le groupe β-cyclodextrine est lié au support via éventuellement un groupe espaceur.

7. Biocapteur selon la revendication 6, dans lequel le groupe β-cyclodextrine répond à la formule (III) suivants : 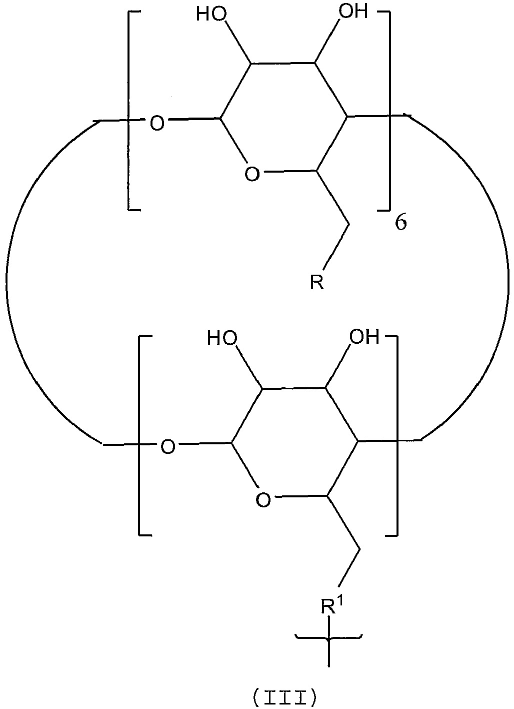 dans laquelle R et R¹ répondent à la même définition que celle donnée à la revendication 6.

8. Biocapteur selon la revendication 4, dans lequel le groupe espaceur est un groupe hydrocarboné, sous forme d'une chaîne linéaire ou ramifiée, pouvant comporter un ou plusieurs groupes aromatiques et pouvant comporter un ou plusieurs hétéroatomes, tels que O, S et N.

9. Biocapteur selon la revendication 4, dans lequel le groupe espaceur est une chaîne hydrocarbonée linéaire interrompue par un ou plusieurs hétéroatomes, ladite chaîne hydrocarbonée pouvant être terminée par un groupe terminal non hydrocarboné assurant la jonction avec le groupe formant cage.

10. Biocapteur selon la revendication 4, dans lequel le groupe espaceur est une chaîne hydrocarbonée ramifiée comportant une chaîne principale hydrocarbonée et un ou plusieurs groupes pendants à ladite chaîne principale, ce ou ces groupes pendants assurant la liaison avec le ou les groupes formant cage, ladite chaîne principale pouvant être liée directement au support ou via un groupe formant pont entre ladite chaîne principale et ledit support.

11. Biocapteur selon l'une quelconque des revendications précédentes, dans lequel la protéase est une enzyme MMP.

12. Biocapteur selon l'une quelconque des revendications précédentes, dans lequel le ou les groupes appartenant au composé comprenant une séquence peptidique cible de la protéase, dont on veut déterminer la présence et/ou l'activité enzymatique, apte(s) à se complexer avec le groupe formant cage, sont des groupes cycliques hydrocarbonés.

13. Biocapteur selon l'une quelconque des revendications précédentes, dans lequel le composé comprenant une séquence peptidique cible de la ou les protéases dont on veut déterminer la présence et/ou l'activité enzymatique répond à la formule (VII) suivant
(X₁)ₙ-S-(X₂)ₘ (VII)
dans laquelle S correspond à une séquence peptidique cible de la protéase (dont on veut déterminer la présence et/ou l'activité enzymatique);
- n et m sont des entiers égaux à 0 ou 1, étant entendu que l'un au moins des n et m est différent de zéro ;
- lorsque m et n sont égaux à 1, X₁ est une sonde porteuse d'un groupe donneur fluorescent et X₂ est une sonde porteuse d'un groupe accepteur fluorescent ou non fluorescent, le groupe donneur et le groupe accepteur étant choisis, de préférence, de sorte à ce que le spectre d'émission de fluorescence du groupe donneur recouvre, au moins en partie, le spectre d'absorption du groupe accepteur, sachant que l'un au moins des X₁ ou X₂ comporte un groupe apte à se complexer avec un groupe formant cage tel que mentionné à la revendication 1.

14. Biocapteur selon la revendication 13, dans lequel le groupe donneur fluorescent porté par X₁ et le groupe accepteur fluorescent ou non fluorescent porté par X₂ sont des groupes coumarines, lesquels groupes sont aptes à former un complexe avec un groupe formant cage tel que mentionné à la revendication 1.

15. Biocapteur selon l'une quelconque des revendications précédentes, dans lequel le composé comprenant une séquence peptidique cible de la ou les protéases dont on veut déterminer la présence et/ou l'activité enzymatique répond à la formule (VII) suivant
A-NH-D (CH₂-C(=O)-R₂-B)₂ (VII)
dans laquelle :
A est un groupe comprenant une séquence peptidique cible de la protéase dont on veut déterminer la présence et/ou l'activité enzymatique;
B est un groupe adamantane;
D représente un atome d'azote ; et
R₂ représente une liaison simple ou un groupement de liaison comprenant de 1 à 40 atomes de carbone.

## Patentansprüche

1. Biosensor zur Erfassung des Vorhandenseins und/oder der enzymatischen Aktivität wenigstens einer Protease, entsprechend einem Biosensor umfassend einen Träger, der funktionalisiert ist durch organische Verbindungen, die wenigstens eine käfigbildende Gruppe umfassen, welche käfigbildende Gruppe mit wenigstens einer Gruppe einer Verbindung komplexiert ist, die eine Peptidsequenz umfasst, die Ziel der Protease(n) ist, deren Vorhandensein und/oder enzymatische Aktivität man bestimmen möchte, wobei der Biosensor regenerierbar ist.

2. Biosensor nach Anspruch 1, bei dem der Träger ein fester anorganischer Träger ist.

3. Biosensor nach Anspruch 2, bei dem der feste anorganische Träger ein Material umfasst ausgewählt aus der Gruppe gebildet durch die Gläser, die Quarze, die Keramiken, die Metalle und die Metalloide.

4. Biosensor nach einem der vorhergehenden Ansprüche, bei dem die organischen Verbindungen aus einem Spacerarm bestehen, der an einem seiner Enden mit dem Träger und mit wenigstens einem anderen seiner Enden mit wenigstens einer käfigbildenden Gruppe verbunden ist.

5. Biosensor nach Anspruch 4, bei dem die käfigbildende Gruppe eine *β*-Cyclodextringruppe ist.

6. Biosensor nach Anspruch 5, bei dem die *β*-Cyclodextringruppe eine zyklische Gruppe ist, die in ihrem Ring wenigstens ein sich wiederholendes Motiv der nachfolgenden Formel (I) umfasst: 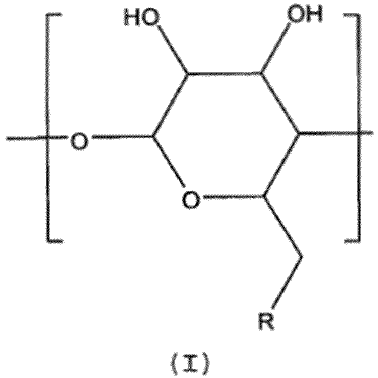 wobei R eine Hydroxylgruppe oder eine Aminogruppe darstellt,
sowie wenigstens ein Motiv der nachfolgenden Formel (II) 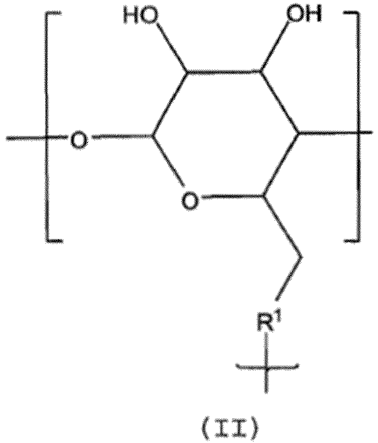 wobei R¹ einem Sauerstoffatom oder einer Aminogruppe entspricht,
wobei die geschweifte Klammer den Ort angibt, an dem die *β-*Cyclodextringruppe mit dem Träger verbunden ist, gegebenenfalls über eine Spacergruppe.

7. Biosensor nach Anspruch 6, bei dem die *β*-Cyclodextringruppe der nachfolgenden Formel (III) genügt: 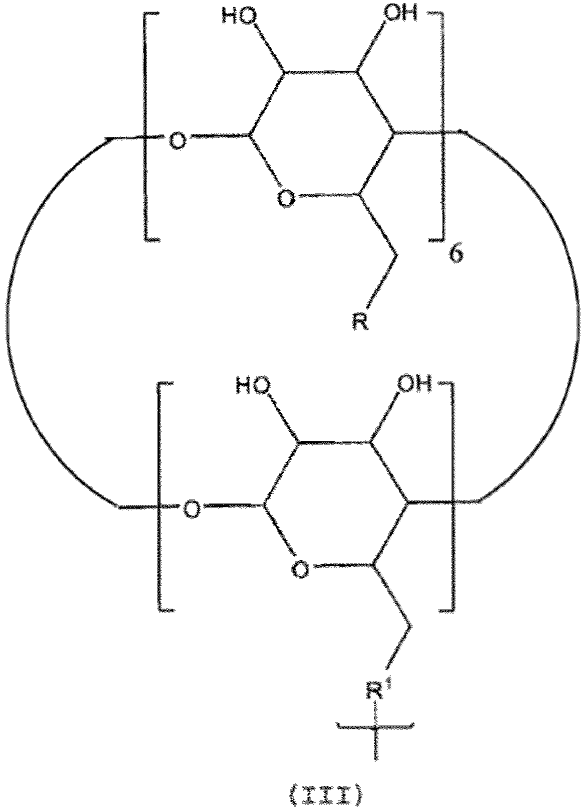 wobei R und R¹ der gleichen Definition wie der in Anspruch 6 angegebenen entsprechen.

8. Biosensor nach Anspruch 4, bei dem die Spacergruppe eine Kohlenwasserstoffgruppe in Form einer linearen oder verzweigten Kette ist, die eine oder mehrere aromatische Gruppen umfassen kann, und die ein oder mehrere Heteroatome umfassen kann, wie zum Beispiel O, S und N.

9. Biosensor nach Anspruch 4, bei dem die Spacergruppe eine lineare Kohlenwasserstoffkette ist, die durch ein oder mehrere Heteroatome unterbrochen ist, wobei die Kohlenwasserstoffkette durch eine Nicht-Kohlenwasserstoff-Endgruppe beendet sein kann, die die Verbindung mit der käfigbildenden Gruppe gewährleistet.

10. Biosensor nach Anspruch 4, bei dem die Spacergruppe eine verzweigte Kohlenwasserstoffkette ist, umfassend eine Kohlenwasserstoff-Hauptkette und eine oder mehrere an der Hauptkette angehängte Gruppen, wobei diese angehängte Gruppe(n) die Verbindung mit der oder den käfigbildenden Gruppe(n) gewährleistet/gewährleisten, wobei die Hauptkette direkt mit dem Träger verbunden sein kann oder über eine Gruppe, die eine Brücke zwischen der Hauptkette und dem Träger bildet.

11. Biosensor nach einem der vorhergehenden Ansprüche, bei dem die Protease ein MMP-Enzym ist.

12. Biosensor nach einem der vorhergehenden Ansprüche, bei dem die Gruppe(n), die zu der Verbindung gehört/gehören, welche eine Peptidsequenz umfasst, die Ziel der Protease ist, deren Vorhandensein und/oder enzymatische Aktivität man bestimmen möchte, und die dazu ausgelegt ist/sind, sich mit der käfigbildenden Gruppe zu komplexieren, zyklische Kohlenwasserstoffgruppen sind.

13. Biosensor nach einem der vorhergehenden Ansprüche, bei dem die Verbindung, die eine Peptidsequenz umfasst, welche Ziel der Protease(n) ist, deren Vorhandensein und/oder enzymatische Aktivität man bestimmen möchte, der nachfolgenden Formel (VII) genügt:
(X₁)ₙ-S-(X₂)ₘ (VII)
wobei S einer Peptidsequenz entspricht, die Ziel der Protease ist (deren Vorhandensein und/oder enzymatische Aktivität man bestimmen möchte);
- n und m ganze Zahlen gleich 0 oder 1 sind, wobei es sich versteht, dass wenigstens eine von n und m von 0 verschieden ist;
- wenn m und n gleich 1 sind, X₁ eine Sonde ist, die Träger einer fluoreszierenden Donorgruppe ist, und X₂ eine Sonde ist, die Träger einer fluoreszierenden oder nicht fluoreszierenden Akzeptorgruppe ist, wobei die Donorgruppe und die Akzeptorgruppe vorzugsweise derart ausgewählt sind, dass das Fluoreszenzemissionsspektrum der Donorgruppe wenigstens teilweise das Absorptionsspektrum der Akzeptorgruppe überdeckt, wobei wenigstens eines von X₁ oder X₂ eine Gruppe umfasst, die dazu ausgelegt ist, sich mit einer käfigbildenden Gruppe wie in Anspruch 1 genannt zu komplexieren.

14. Biosensor nach Anspruch 13, bei dem die von X₁ getragene fluoreszierende Donorgruppe und die von X₂ getragene fluoreszierende oder nicht fluoreszierende Akzeptorgruppe Kumaringruppen sind, welche Gruppen dazu ausgelegt sind, mit einer käfigbildenden Gruppe wie in Anspruch 1 genannt einen Komplex zu bilden.

15. Biosensor nach einem der vorhergehenden Ansprüche, bei dem die Verbindung, die eine Peptidsequenz umfasst, welche Ziel der Protease(n) ist, deren Vorhandensein und/oder enzymatische Aktivität man bestimmen möchte, der nachfolgenden Formel (VII) genügt:
A-NH-D(CH₂-C(=O)-R₂-B)₂ (VII)
wobei:
A eine Gruppe ist, die eine Peptidsequenz umfasst, welche Ziel der Protease ist, deren Vorhandensein und/oder enzymatische Aktivität man bestimmen möchte;
B eine Adamantangruppe ist;
D ein Stickstoffatom darstellt; und
R₂ eine einfache Bindung oder eine Bindungsgruppe darstellt, die 1 bis 40 Kohlenstoffatome umfasst.

## Claims

1. A biosensor for detecting the presence and/or the enzyme activity of at least one protease, which corresponds to a biosensor comprising a support functionalized by organic compounds comprising at least one cage-forming group, which cage-forming group is complexed with at least one group of a compound comprising a target peptide sequence of the protease(s) the presence and/or enzyme activity of which are to be determined, which biosensor is regenerable.

2. The biosensor according to claim 1, wherein the support is an inorganic solid support.

3. The biosensor according to claim 2, wherein the inorganic solid support comprises a material selected from the group consisting of glasses, quartz, ceramics, metals and metalloids.

4. The biosensor according to any of the preceding claims, wherein the organic compounds consist in a spacer arm linked at one of its ends to the support and at at least another of its ends to at least one cage-forming group.

5. The biosensor according to claim 4, wherein the cage-forming group is a β-cyclodextrin group.

6. The biosensor according to claim 4, wherein the β-cyclodextrine group is a ring group comprising, in each ring, at least one repeating unit having the following formula (I): 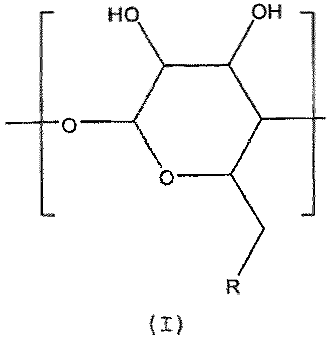 wherein R represents a hydroxyl group or an amino group,
and at least one unit having the following formula (II): 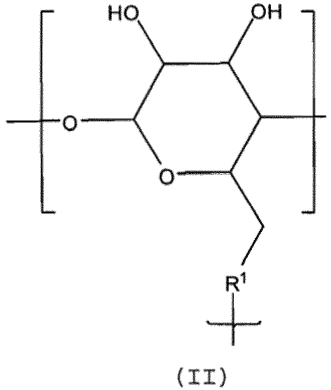 R¹ corresponding to an oxygen atom or to an amino group,
the brace indicating the place at which the β-cyclodextrin group is linked to the support possibly via a spacer group.

7. The biosensor according to claim 6, wherein the β-cyclodextrine group has the following formula (III): 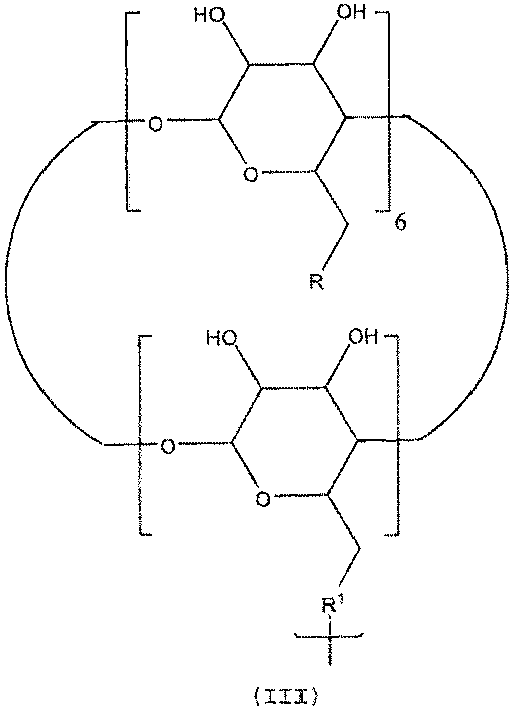 wherein R and R¹ have the same definition as the one given in claim 6.

8. The biosensor according to claim 4, wherein the spacer group is a hydrocarbon group, as a linear or branched chain, that can include one or more aromatic groups and one or more heteroatoms, such as O, S and N.

9. The biosensor according to claim 4, wherein the spacer group is a linear hydrocarbon chain interrupted by one or several heteroatoms, said hydrocarbon chain can be ended by a non-hydrocarbon terminal group providing the junction with the cage-forming group.

10. The biosensor according to claim 4, wherein the spacer group is a branched hydrocarbon chain including a main hydrocarbon chain and one or more pendant groups to said main chain, this/these pendant groups providing linking with the cage-forming group(s), said main chain can be directly linked to the support or via a bridge-forming group between said main chain and said support.

11. The biosensor according to any of the preceding claims, wherein the protease is an MMP enzyme.

12. The biosensor according to any of the preceding claims, wherein the group(s) belonging to the compound comprising a target peptide sequence of the protease, the presence and/or enzyme activity of which are to be determined, able to complex with the cage-forming group, are hydrocarbon ring groups.

13. The biosensor according to any of the preceding claims, wherein the compound comprising a target peptide sequence of the protease(s), the presence and/or enzyme activity of which are to be determined, has the following formula (VII):
(X₁)ₙ-S-(X₂)ₘ (VII)
wherein S corresponds to a target peptide sequence of the protease (the presence and/or the enzyme activity of which are to be determined);
- n and m are integers equal to 0 or 1, given that at least one of n and m is different from zero;
- when m and n are equal to 1, X₁ is a probe carrying a fluorescent donor group and X₂ is a probe carrying a fluorescent or non-fluorescent acceptor group, the donor group and the acceptor group being preferably selected such that the fluorescence emission spectrum of the donor group at least partly covers the absorption spectrum of the acceptor group, with the proviso that at least one of X₁ or X₂ includes a group able to be complexed with a cage-forming group such as mentioned in claim 1.

14. The biosensor according to claim 13, wherein the fluorescent donor group carried by X₁ and the fluorescent or non-fluorescent acceptor group carried by X₂ are coumarin groups, which groups are able to form a complex with a cage-forming group such as mentioned in claim 1.

15. The biosensor according to any of the preceding claims, wherein the compound comprising a target peptide sequence of the protease(s) the presence and/or the enzyme activity of which are to be determined has the following formula (VII):
A-NH-D(CH₂-C(=O)-R₂-B)₂ (VII)
wherein:
A is a group comprising a target peptide sequence of the protease the presence and/or the enzyme activity of which are to be determined;
B is an adamantane group;
D represents a nitrogen atom; and
R₂ represents a single bond or a binding group comprising from 1 to 40 carbon atoms.
